# FASCICULE DE BREVET EUROPEEN

(11) **EP 0 920 856 B1**
(45) Date de publication et mention de la délivrance du brevet: **28.01.2004**
(21) Numéro de dépôt: 98402901.7
(22) Date de dépôt: 20.11.1998
(51) Int. Cl.: A61K 7/13, D06B 3/18, D06P 5/13

(54) **Procédé de teinture directe en deux étapes des fibres kératiniques mettant en oeuvre des colorants directs basiques**
Verfahren zur Direkt-Färbung von Keratin-Fasern in zwei Stufen mit Hilfe von Direct Basic Färbungsmitteln
Process for direct dying of keratinous fibres in two steps with the help of direct basic dyes

(30) Priorité: 05.12.1997 FR 9715413
(43) Date de publication de la demande: 09.06.1999
(73) Titulaire: L'OREAL, 75008 Paris (FR)
(72) Inventeur: Deneulenaere, Christelle, 78110 Le Vesinet (FR)
(74) Mandataire: Miszputen, Laurent

(56) Documents cités:
- FR-A- 1 585 308
- US-A- 3 679 347

## Description

L'invention concerne un procédé de teinture directe, en deux temps, des fibres kératiniques, en particulier des fibres kératiniques humaines telles que les cheveux, caractérisé par le fait que dans une première étape, on procède à une décoloration des fibres kératiniques, puis dans une deuxième étape, on applique sur les fibres kératiniques une composition tinctoriale contenant, dans un milieu aqueux, au moins un colorant direct basique partiellement dissous, ladite composition tinctoriale étant prête à l'emploi ou résultant du mélange extemporané d'au moins une composition pulvérulente (P) contenant au moins un colorant direct basique et d'une composition aqueuse (A).

Il est bien connu de teindre les fibres kératiniques et en particulier les cheveux humains avec des compositions tinctoriales contenant des colorants directs et en particulier des colorants directs nitrés benzéniques. Les colorants directs ont cependant l'inconvénient, lorsqu'ils sont incorporés dans des compositions tinctoriales, de conduire à des colorations présentant une ténacité insuffisante, en particulier vis-à-vis des shampooings. De plus, les colorations obtenues manquent en général de puissance et sont trop ternes.

Afin de remédier à ce problème, il a déjà été proposé de procéder à une décoloration des fibres kératiniques préalablement à toute étape d'application des colorants directs, notamment dans le brevet US 3,679,347. Cependant l'application des colorants directs sur des fibres kératiniques décolorées, bien que conduisant à des colorations moins ternes que celles obtenues sans procéder à une décoloration préalable des fibres, conduit cependant à des colorations insuffisamment chromatiques et puissantes et non homogènes, c'est à dire présentant des différences locales d'intensité.

C'est en cherchant à résoudre ces problèmes que la Demanderesse vient maintenant de découvrir de manière surprenante qu'il est possible d'obtenir d'obtenir des teintures puissantes, très chromatiques et homogènes, présentant en outre une bonne résistance vis-à-vis des agents atmosphériques tels que la lumière et les intempéries, et vis-à-vis de la transpiration et des différents traitements que peuvent subir les cheveux (lavages, déformations permanentes), en mettant en oeuvre un procédé de teinture directe en deux temps des fibres kératiniques consistant dans une première étape à réaliser une décoloration des fibres kératiniques, puis dans une deuxième étape, à appliquer sur ces fibres, une composition tinctoriale contenant, dans un milieu aqueux, au moins un colorant direct basique partiellement dissous, ladite composition tinctoriale étant prête à l'emploi ou résultant du mélange extemporané d'au moins une composition pulvérulente (P) contenant au moins un colorant direct basique et d'une composition aqueuse (A).

Le procédé de teinture conforme à l'invention permet également de teindre les fibres kératiniques avec de faibles temps de pause.

Cette découverte est à la base de la présente invention.

La présente invention a donc pour objet un procédé de teinture directe en deux étapes des fibres kératiniques, et en particulier des fibres kératiniques humaines telles que les cheveux, caractérisé par le fait que dans une première étape, on procède à une décoloration des fibres kératiniques, puis dans une deuxième étape, on applique sur les fibres kératiniques une composition tinctoriale contenant, dans un milieu aqueux, au moins un colorant direct basique partiellement dissous, ladite composition tinctoriale étant prête à l'emploi ou résultant du mélange extemporané d'au moins une composition pulvérulente (P) contenant au moins un colorant direct basique et d'une composition aqueuse (A).

Selon l'invention, le fait que le ou les colorants directs basiques présents dans la composition tinctoriale appliquée lors de la deuxième étape du procédé conforme à l'invention soient partiellement dissous signifie soit que le ou les colorants directs basiques sont en sursaturation, c'est à dire présents en une quantité pondérale supérieure à leur limite de solubilité dans le milieu tinctorial aqueux utilisé, soit que le ou les colorants directs basiques sont partiellement ou totalement adsorbés sur ou enrobés par une charge minérale ou organique insoluble présente dans la composition tinctoriale.

Selon l'invention, la première étape du procédé conforme à l'invention est une étape de décoloration conduisant à une nuance, (après décoloration), présentant de préférence une hauteur de ton supérieure ou égale à 6.

Dans le domaine de la teinture des fibres kératiniques, la couleur des fibres kératiniques peut être exprimée en hauteurs de tons allant de 1 à 10 et correspondant aux nuances suivantes :

| **Hauteur de tons** | **Nuance correspondante** |
|---|---|
| 10 | Blond très très clair |
| 9 | Blond très clair |
| 8 | Blond clair |
| 7 | Blond |
| 6 | Blond foncé |
| 5 | Châtain clair |
| 4 | Châtain |
| 3 | Châtain foncé |
| 2 | Brun |
| 1 | Noir |

De préférence, l'étape de décoloration conduit à une nuance présentant une différence de couleur supérieure ou égale à 2 tons par rapport à la couleur des fibres avant la décoloration. Les résultats sont d'autant meilleurs que l'on s'approche, après l'étape de décoloration, des hauteurs de tons 9 ou 10.

Tous les types de méthodes de décoloration des fibres kératiniques peuvent être utilisés selon le procédé de l'invention.

Selon une première forme de réalisation du procédé de l'invention, la décoloration peut être effectuée par application d'une composition oxydante contenant au moins un agent oxydant.

Le temps nécessaire à l'obtention de la décoloration désirée est en général compris entre 15 et 60 minutes et encore plus particulièrement entre 30 et 45 minutes.

La nature de l'agent oxydant présent dans la composition oxydante n'est pas critique. Parmi ces agents oxydants, on peut notamment citer le peroxyde d'hydrogène, le peroxyde d'urée, les bromates de métaux alcalins, les persels tels que les perborates, les persulfates et les percarbonates, les polythionates et leurs mélanges.

La composition oxydante peut se présenter sous forme liquide ou crémeuse, ladite composition oxydante étant prête à l'emploi ou résultant du mélange extemporané d'une ou plusieurs compositions aqueuses ou d'une ou plusieurs compositions aqueuses avec une ou plusieurs compositions pulvérulentes, l'agent oxydant étant présent dans la ou les compositions aqueuses et/ou dans la ou les compositions pulvérulentes.

Lorsque l'agent oxydant est présent dans la composition pulvérulente, celle-ci peut alors se présenter sous forme granulée ou enrobée tel que cela est décrit par exemple dans les demandes de brevets FR-A-2 703 588, FR-A-2 703 589, FR-A-2 715 065, FR-A-2 716 804 au nom de la Demanderesse,

Le pH de la composition oxydante est de préférence compris entre 5 et 12 et encore plus particulièrement entre 8 et 11, et peut être ajusté à la valeur désirée au moyen d'agents acidifiants ou alcalinisants bien connus de l'état de la technique en décoloration des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés décrits dans la demande de brevet EP-A-512 879 au nom de la Demanderesse et parmi lesquels on peut notamment citer le 1,3-diaminopropane, le N,N'-diéthyl 1,3-diaminopropane, le N,N-diéthyl 1,3-diaminopropane, le N,N-diméthyl 1,3-diaminopropane, le 2-hydroxy 1-(N,N-diéthyl)amino 3-aminopropane, et le 2-hydroxy 1,3-diaminopropane.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition oxydante utilisée selon le procédé conforme à l'invention peut en outre renfermer un ou plusieurs adjuvants utilisés classiquement dans les compositions pour la décoloration des fibres kératiniques, tels que des agents tensio-actifs, des polymères, des agents épaississants minéraux ou organiques, des agents antioxydants, des agents de pénétration, des agents séquestrants, des parfums, des tampons, des agents dispersants, des agents de conditionnement, des agents filmogènes, des céramides, des agents conservateurs, des agents opacifiants.

Lorsque l'étape de décoloration au moyen de la composition oxydante est terminée, les fibres kératiniques sont de préférence rincées à l'eau avant la deuxième étape d'application de la composition tinctoriale contenant le ou les colorants directs basiques partiellement dissous. Le rinçage peut en outre être suivi d'un shampooing afin d'éliminer les traces éventuelles d'agent oxydant.

Selon une deuxième forme de réalisation de procédé de l'invention, l'étape de décoloration peut être effectuée par irradiation des fibres kératiniques à l'aide d'un rayonnement laser sous forme d'impulsions de puissance suffisante pour dégrader la mélanine contenue dans les fibres kératiniques et ainsi conduire à la décoloration.

Cette méthode de décoloration par irradiation laser est décrite dans les demandes de brevets EP-A-685 220 et EP-A-685 180 de la Demanderesse.

Le ou les colorants directs basiques pouvant être utilisés dans la composition tinctoriale utilisée lors de la deuxième étape du procédé conforme à l'invention sont de préférence choisis parmi les amino-anthraquinones basiques, les mono- ou di-azoïques basiques, les azométhines basiques, les naphtoquinones basiques, et les colorants basiques à mono-insaturations éthyléniques.
A titre d'exemple, on peut notamment citer le chlorure de [8-[(p-aminophényl)azol]-7-hydroxy-2-naphtyl]-triméthylammonium (également appelé Basic Brown 16 ou Arianor Mahogany 306002 dans le Color Index), l'association du chlorure de 3-[(4-amino-6-bromo-5,8-dihydro-1-hydroxy-8-imino-5-oxo-2-naphtyl)-amino]-N,N,N-triméthyl-benzénaminium et du chlorure de 3-[(2,6-dibromo-5,8-dihydro-1-hydroxy-8-imino-5-oxo-3-naphtyl)-amino]-N,N,N-triméthyl-berizénaminium (également dénommé Basic Blue 99 ou Arianor Steel Blue 306004 dans le Color Index), le chlorure de 7-hydroxy-8-[(2-méthoxyphényl)azo]-N,N,N-triméthyl-2-naphtalènaminium (également appelé le Basic Red 76 ou Arianor Madder Red dans le Color Index), le chlorure de [8-[(4-amino-2-nitrophényl)azo]-7-hydroxy-2-naphtyl]triméthylammonium (également appelé Basic Red 118 dans le Color Index), l'association du chlorure de [8-[(4-amino-3-nitrophényl)azo]-7-hydroxy-2-naphtyl]-triméthylammonium et du chlorure de [8-[(4-amino-2-nitrophényl)azo]-7-hydroxy-2-naphtyl]-triméthylammonium (également appelé Brown 17 ou Arianor Sienna Brown 306001 dans le Color Index), le chlorure de 3-[(4,5-dihydro-3-méthyl-5-oxo-1-phényl-1H-pyrazol-4-yl)azo]-N,N,N-triméthyl-benzènaminium (également appelé Basic Yellow 57 ou Arianor Straw Yellow 306005 dans le Color Index), le chlorhydrate de 1-(γ-aminopropyl)amino anthraquinone, le méthylsulfate de 1-N-(méthyl morpholinium propyl)amino 4-hydroxy anthraquinone, et le Basic Orange 69 (dénomination du Color Index).

Le ou les colorants directs basiques peuvent également être choisis parmi :
**a) les composés de formule (I) suivante :** 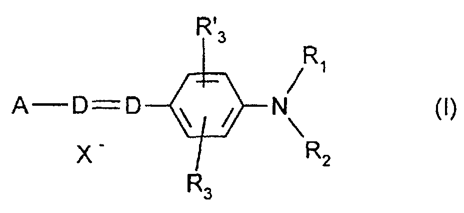 dans laquelle :
   D représente un atome d'azote ou le groupement -CH,
   R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
   X⁻ représente un anion de préférence choisi parmi le chlorure, le méthyl sulfate et l'acétate,
   A représente un groupement choisi par les structures A1 à A19 suivantes : 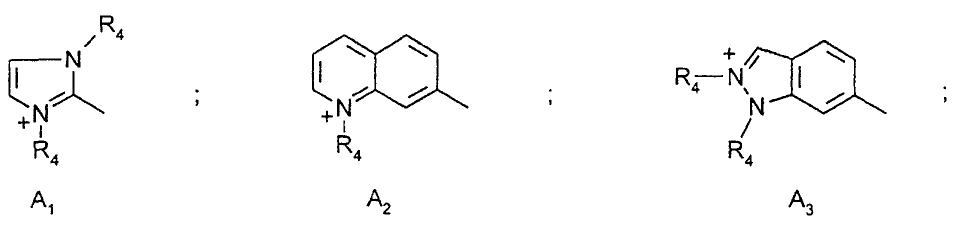 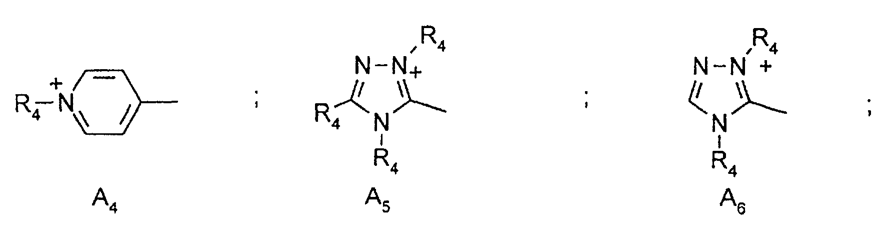 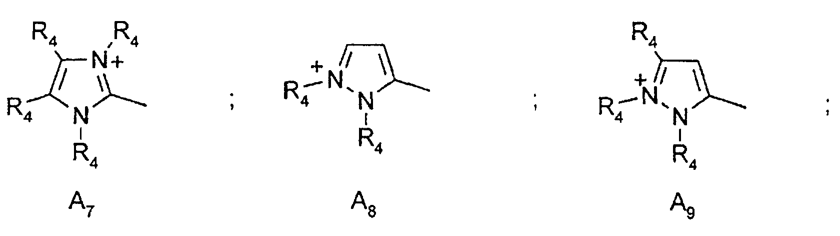 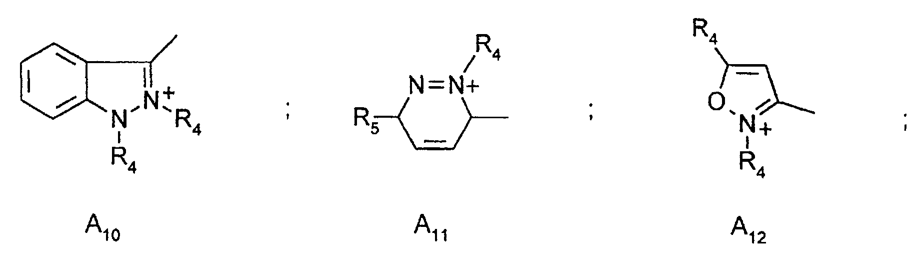 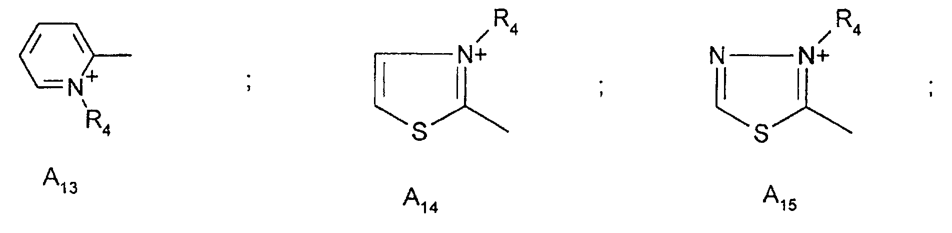 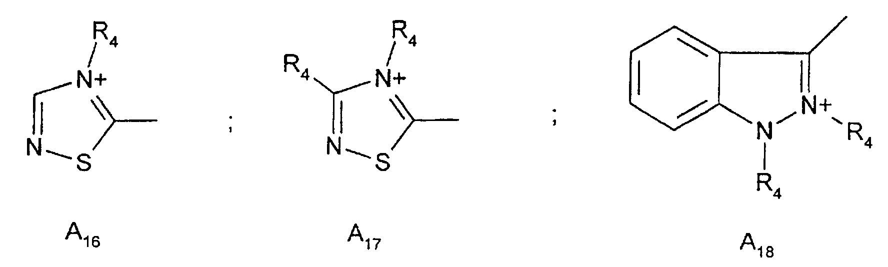 et 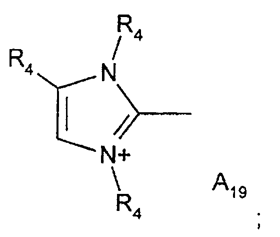 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 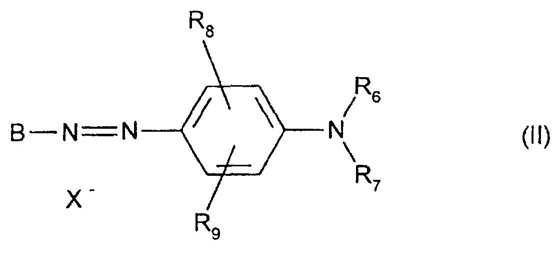 dans laquelle :
   R₆ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C,-C₄ ou alcoxy en C₁-C₄, un radical -CN,
   X⁻ représente un anion de préférence choisi parmi le chlorure, le méthyl sulfate et l'acétate,
   B représente un groupement choisi par les structures B1 à B6 suivantes : 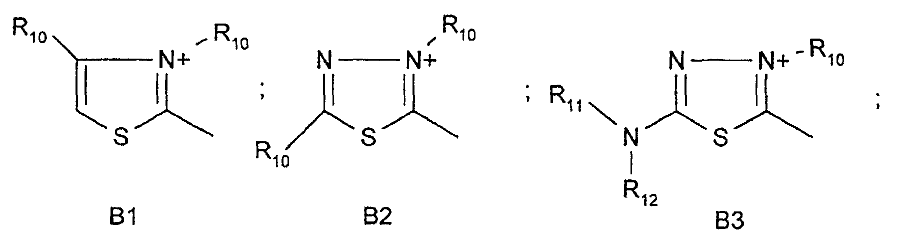 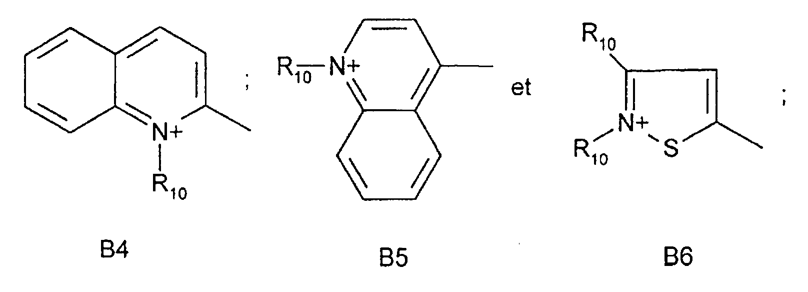 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 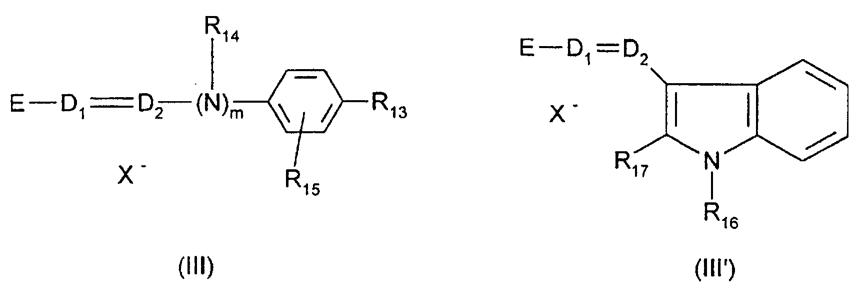 dans lesquelles :
   R₁₃ représente un atome d'hydrogène, un radical alcoxy en C₁-C₄, un atome d'halogène tel que le brome, le chlore, l'iode ou le fluor ou un radical amino,
   R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₁₅ représente un atome d'hydrogène ou d'halogène tel que le brome, le chlore, l'iode ou le fluor,
   R₁₆ et R₁₇, identiques ou différents, représentent un atome d'hydrogène ou un radical alkyle en C₁-C₄,
   D₁ et D₂, identiques ou différents, représentent un atome d'azote ou le groupement -CH,
   m = 0 ou 1,
   étant entendu que lorsque R₁₃ représente un groupement amino non substitué, alors D₁ et D₂ représentent simultanément un groupement -CH et m = 0,
   X⁻ représente un anion de préférence choisi parmi le chlorure, le méthyl sulfate et l'acétate,
   E représente un groupement choisi par les structures E1 à E8 suivantes : 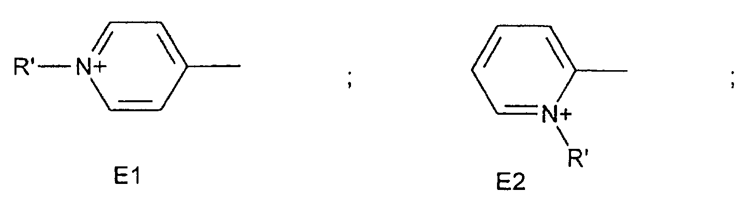 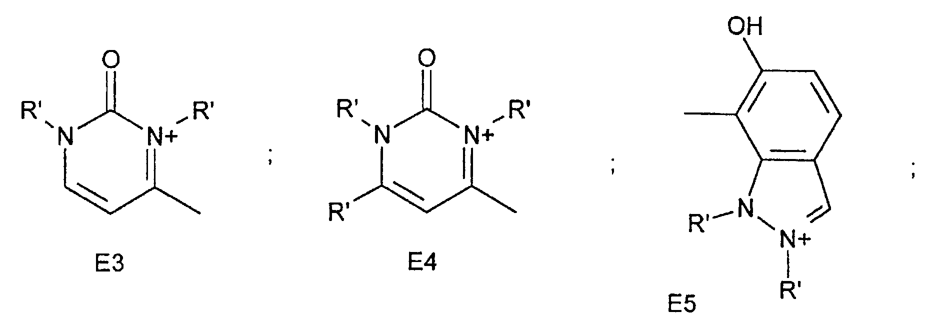 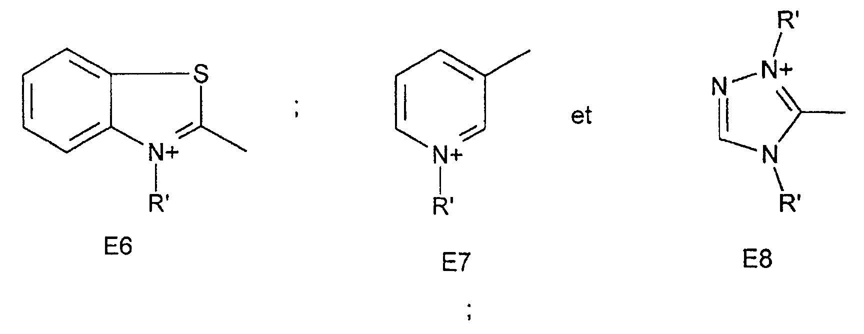 dans lesquelles R' représente un radical alkyle en C₁-C₄ ;
   lorsque m = 0 et que D₁ représente un atome d'azote, alors E peut également désigner un groupement de structure E9 suivante : 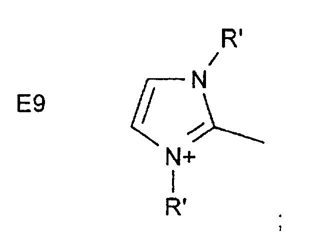 dans laquelle R' représente un radical alkyle en C₁-C₄;
**d) les composés de formule (IV) suivante :**

   **G―N ̿N―J (IV)**

   dans laquelle :
   **le symbole G** représente un groupement choisi parmi les structures G₁ à G₃ suivantes : 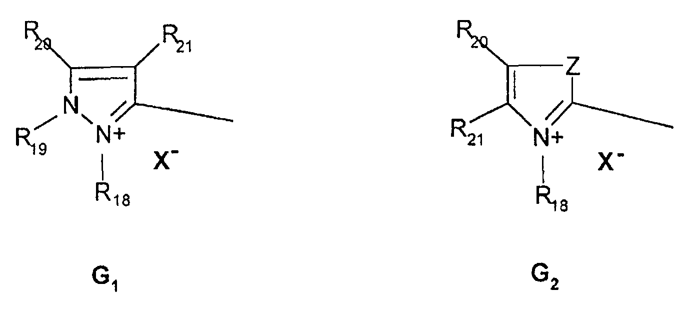 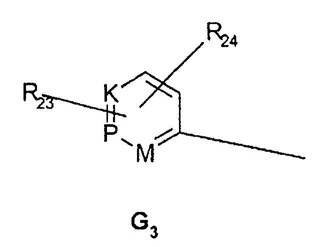 structures G₁ à G₃ dans lesquelles,
      R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
      R₁₉ désigne un radical alkyle en C₁-C₄ ou un radical phényle;
      R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
      R₂₀ peut désigner en outre un atome d'hydrogène;
      Z désigne un atome d'oxygène, de soufre ou un groupement -NR₁₉;
      M représente un groupement -CH, -CR (R désignant alkyle en C₁-C₄), ou -NR₂₂(X⁻)ᵣ;
      K représente un groupement -CH, -CR (R désignant alkyle en C₁-C₄), ou -NR₂₂(X⁻)ᵣ;
      P représente un groupement -CH, -CR (R désignant alkyle en C₁-C₄), ou -NR₂₂(X⁻)ᵣ; r désigne zéro ou 1;
      R₂₂ représente un atome O⁻, un radical alcoxy en C₁-C₄, ou un radical alkyle en C₁-C₄;
      R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
      X⁻ représente un anion de préférence choisi parmi le chlorure, l'iodure, le méthyl sulfate, l'éthyl sulfate, l'acétate et le perchlorate; sous réserve que,
      si R₂₂ désigne O⁻, alors r désigne zéro;
      si K ou P ou M désignent -N-alkyle C₁-C₄ X⁻ , alors R₂₃ ou R₂₄ est différent d'un atome d'hydrogène;
      si K désigne -NR₂₂(X⁻)ᵣ, alors M= P= -CH, -CR;
      si M désigne -NR₂₂(X⁻)ᵣ, alors K= P= -CH, -CR;
      si P désigne -NR₂₂(X⁻)ᵣ, alors K= M et désignent -CH ou -CR;
      si Z désigne un atome de soufre avec R₂₁ désignant alkyle en C₁-C₄ , alors R₂₀ est différent d'un atome d'hydrogène;
      si Z désigne -NR₂₂ avec R₁₉ désignant alkyle en C₁-C₄ , alors au moins l'un des radicaux R₁₈, R₂₀ ou R₂₁ du groupement de structure G₂ est différent d'un radical alkyle en C₁-C₄;
   **le symbole J** représente :
      - **(a)** un groupement de structure J₁ suivante : 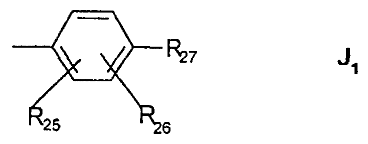 structure J₁ dans laquelle,
         R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
         R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
         R₂₇ représente un atome d'hydrogène, un radical -OH, un radical -NHR₂₈, un radical -NR₂₉R₃₀;
         R₂₈ représente un atome d'hydrogène, un radical alkyle en C₁-C₄, un radical monohydroxyalkyle en C₁-C₄, polyhydroxyalkyle en C₂-C₄, un radical phényle;
         R₂₉ et R₃₀, identiques ou différents, représentent un radical alkyle en C₁-C₄ , un radical monohydroxyalkyle en C₁-C₄, polyhydroxyalkyle en C₂-C₄;
      - **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 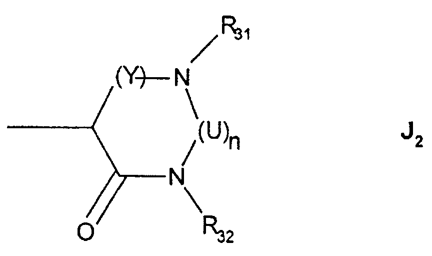 structure J₂ dans laquelle,
         R₃₁ et R₃₂, identiques ou différents, représentent un atome d'hydrogène , un radical alkyle en C₁-C₄, un radical phényle;
         Y désigne le radical -CO- ou le radical 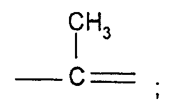 n = 0 ou 1, avec, lorsque n désigne 1, U désigne le radical -CO- .

Dans les structures (I) à (IV) définies ci-dessus le groupement alkyle ou alcoxy en C₁-C₄ désigne de préférence méthyle, éthyle, butyle, méthoxy, éthoxy.

Les colorants directs basiques de formules (I), (II), (III) et (III') utilisables dans la composition tinctoriale utilisée lors de la deuxième étape du procédé conforme à l'invention, sont des composés connus et sont décrits par exemple dans les demandes de brevets WO 95/01772, WO 95/15144 et EP-A-0 714 954.

Parmi les colorants directs basiques de formule (I), on peut plus particulièrement citer les composés répondant aux structures (I1) à (I52) suivantes : 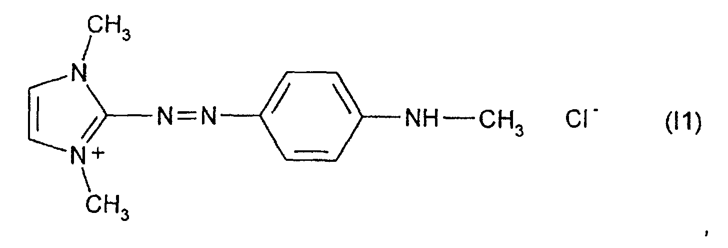 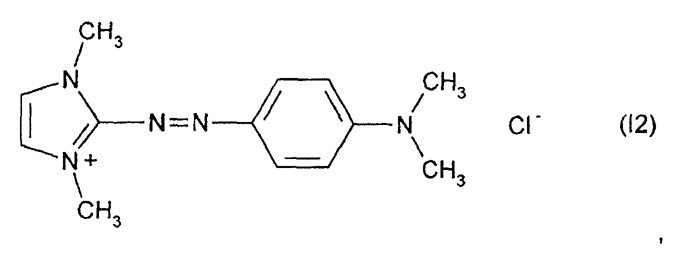 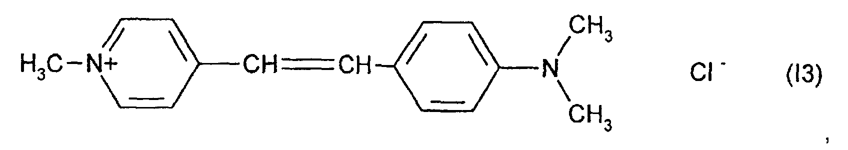 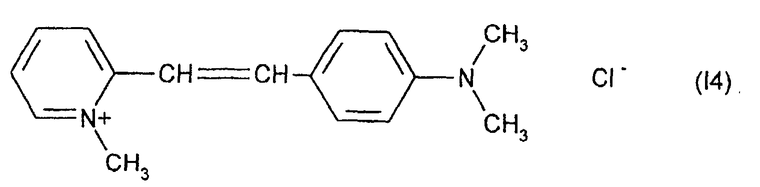 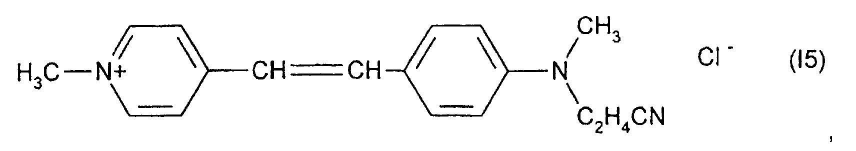 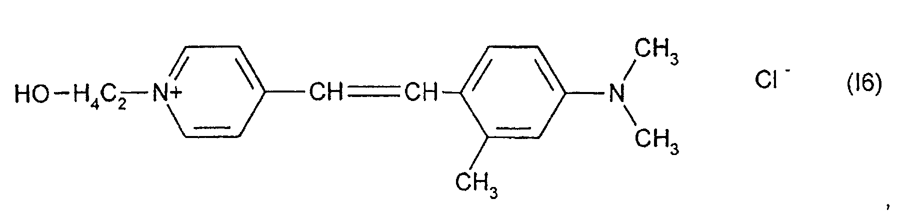 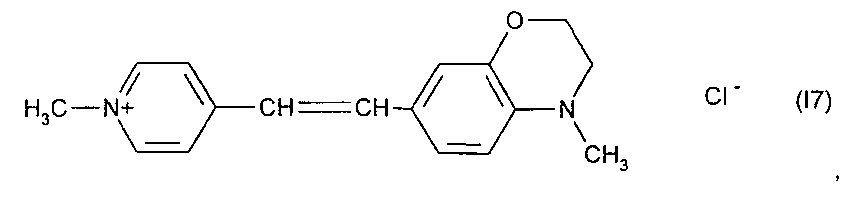 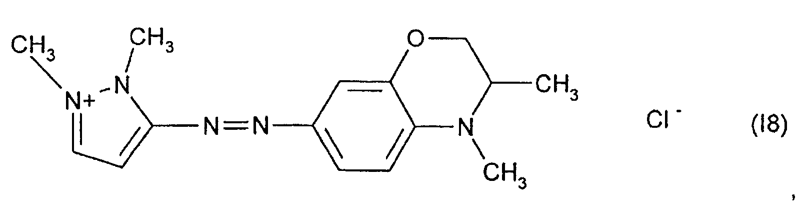 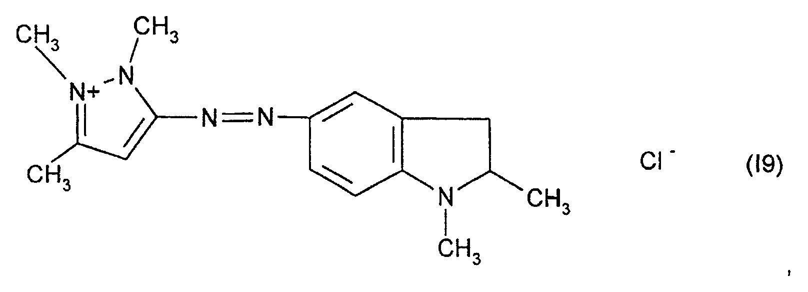 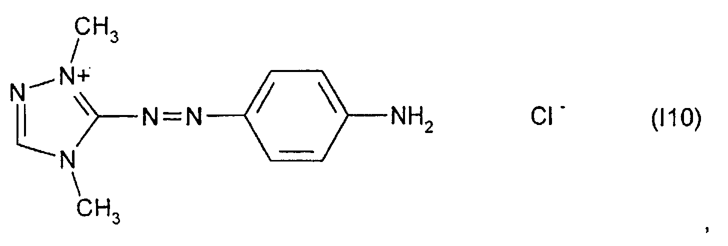 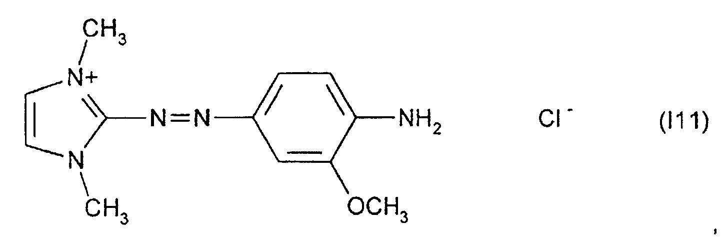 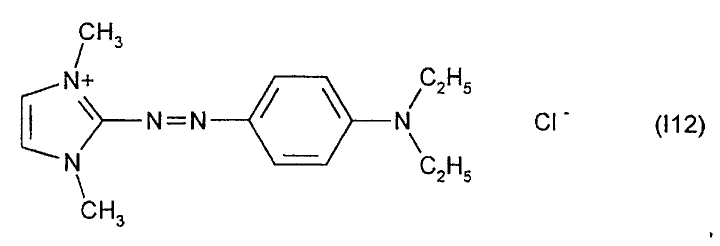 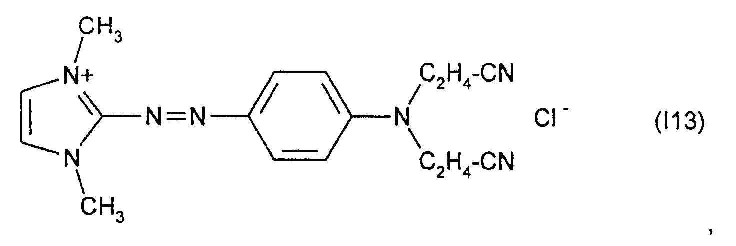 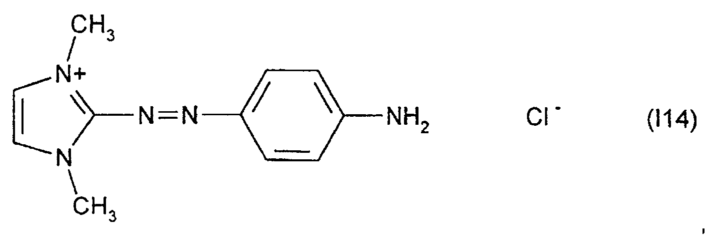 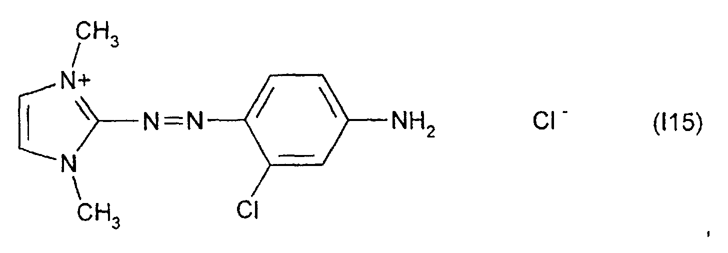 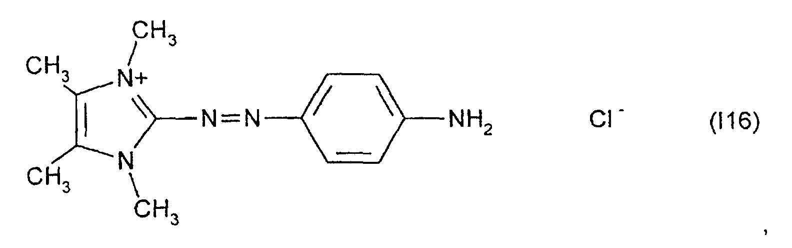 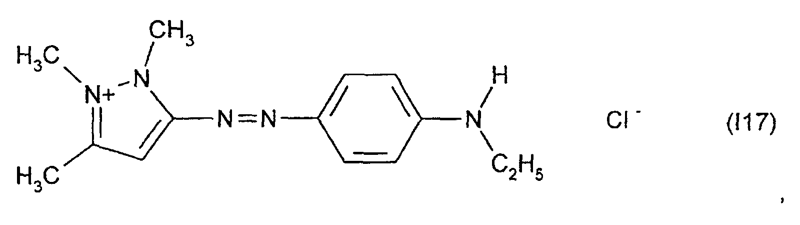 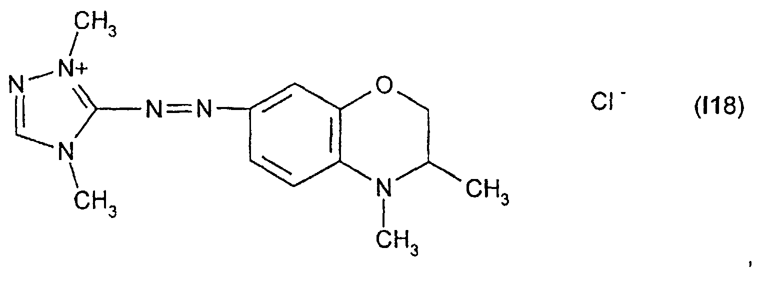 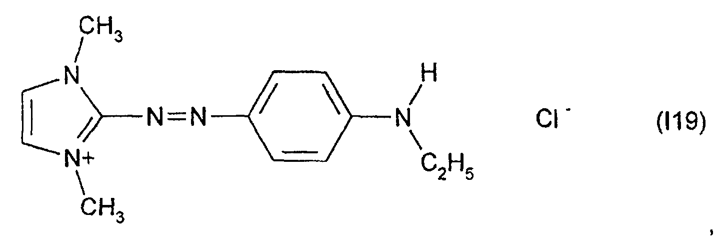 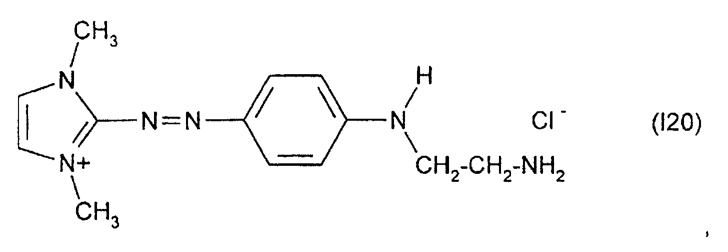 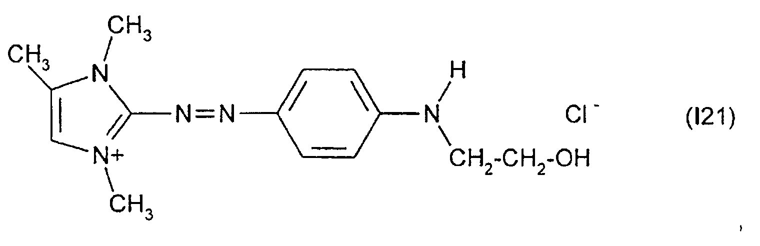 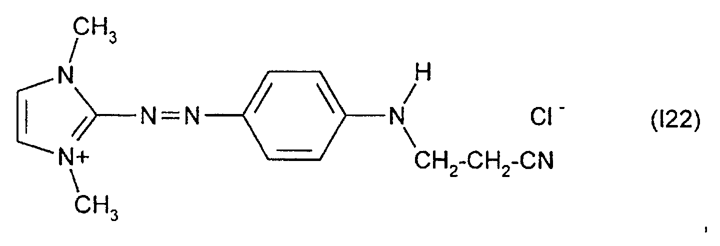 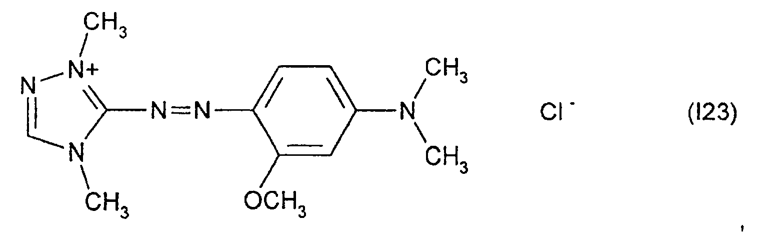 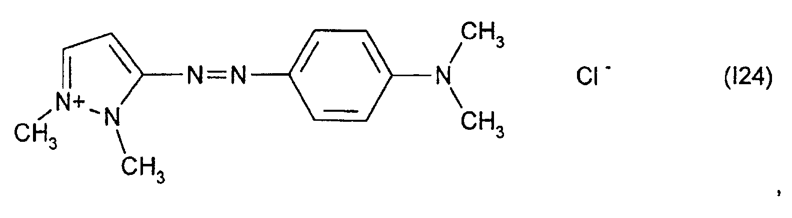 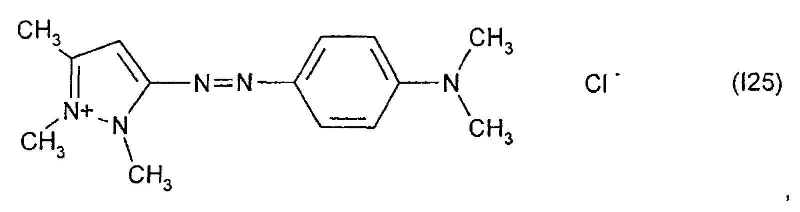 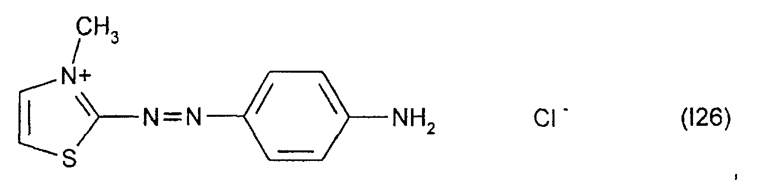 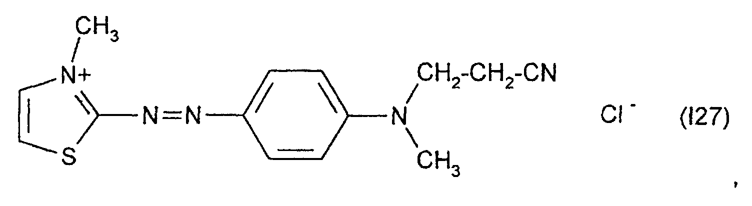 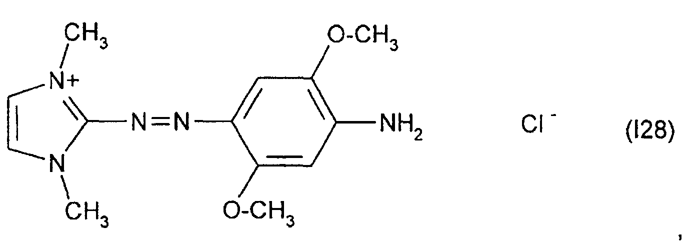 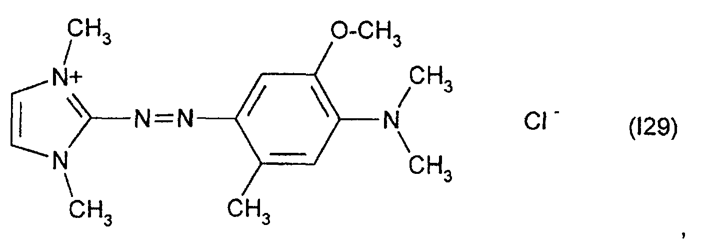 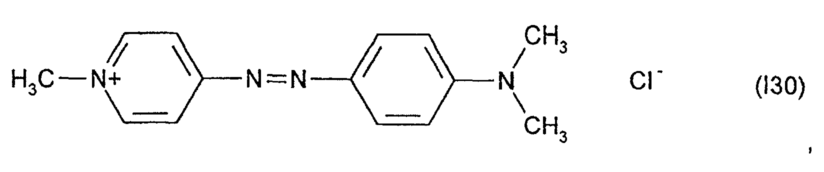 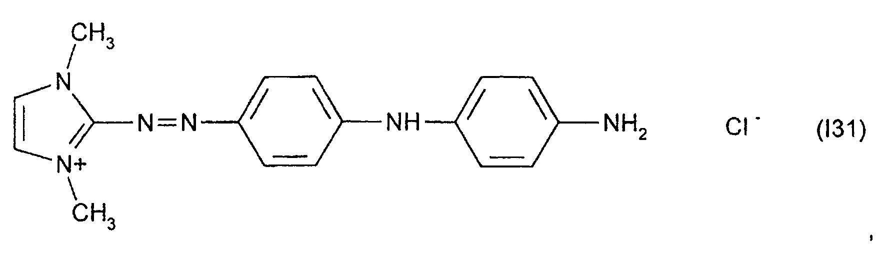 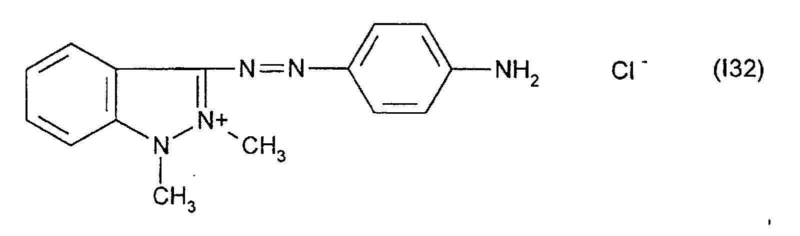 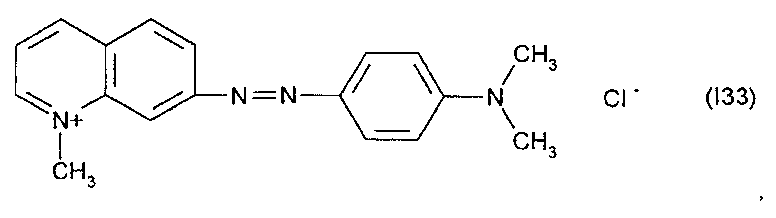 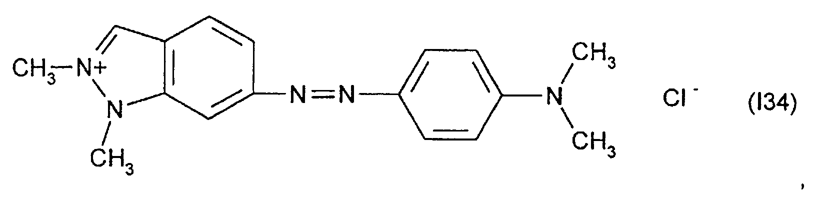 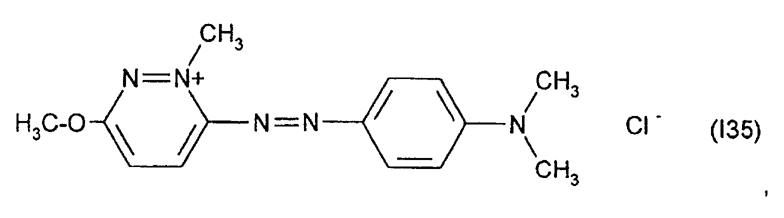 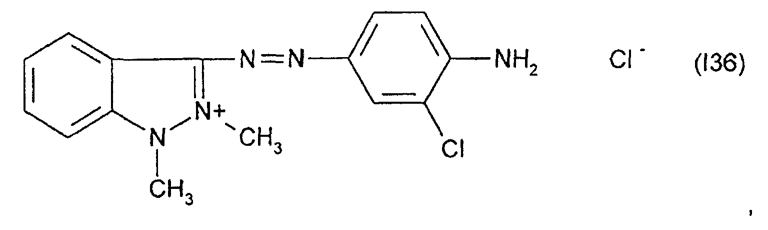 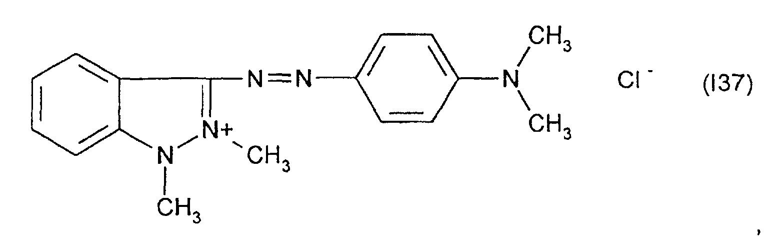 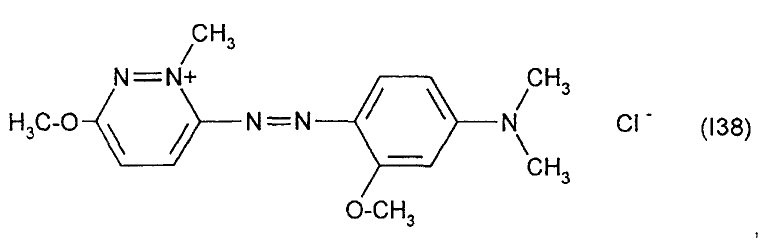 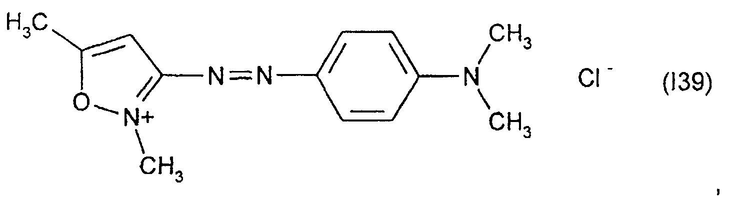 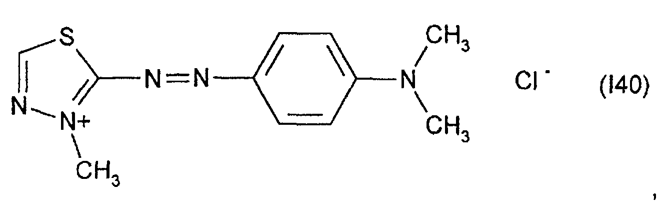 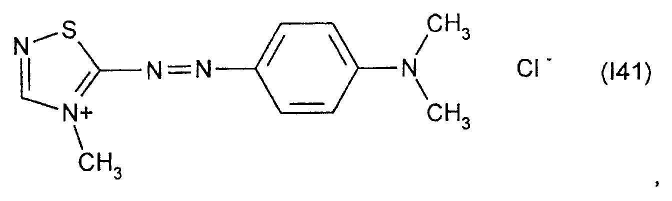 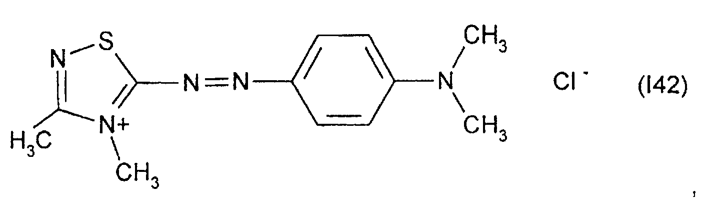 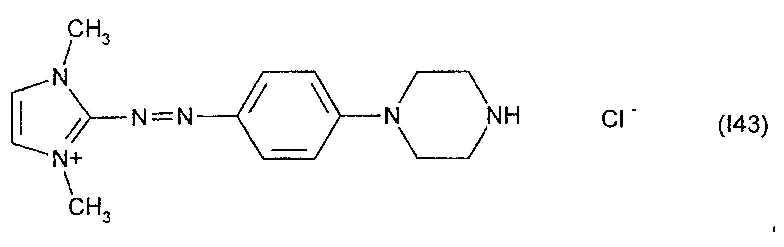 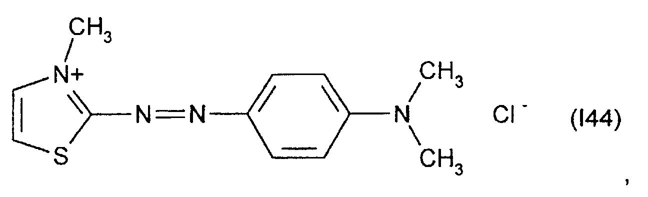 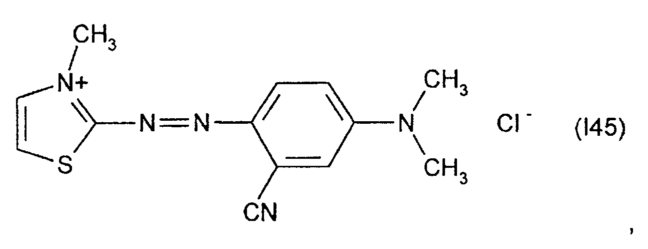 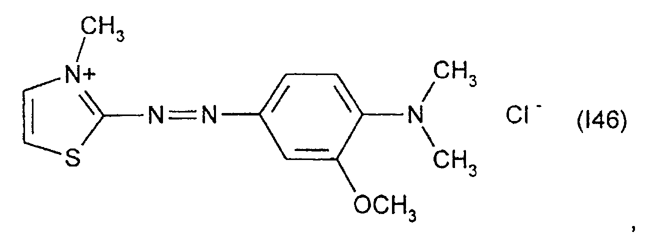 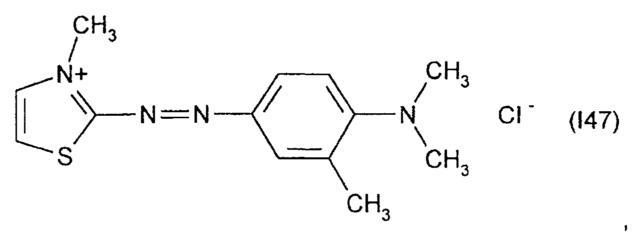 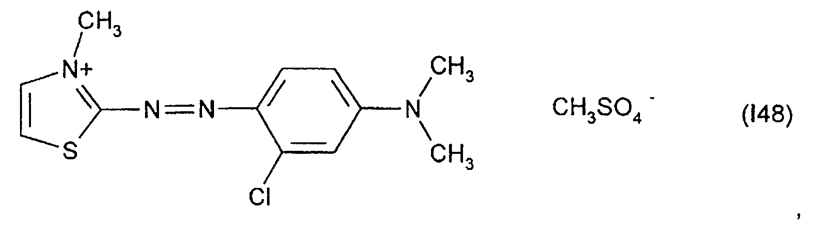 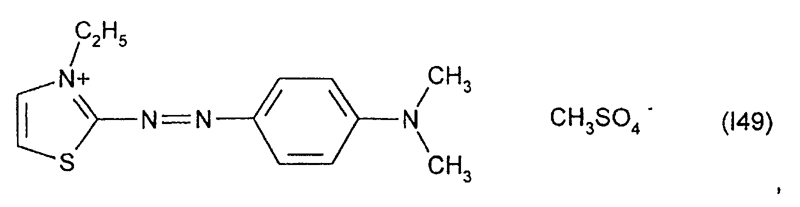 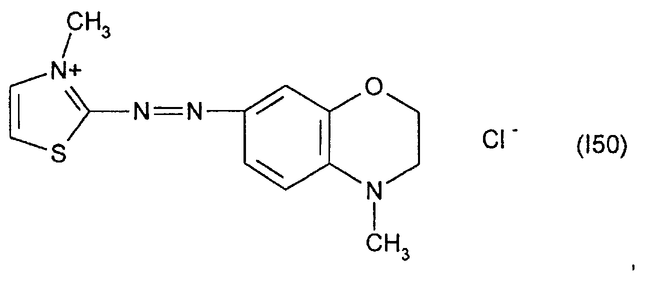 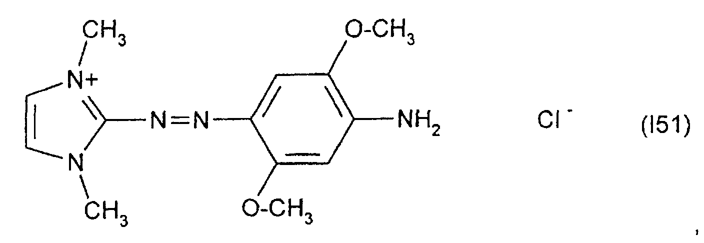 et

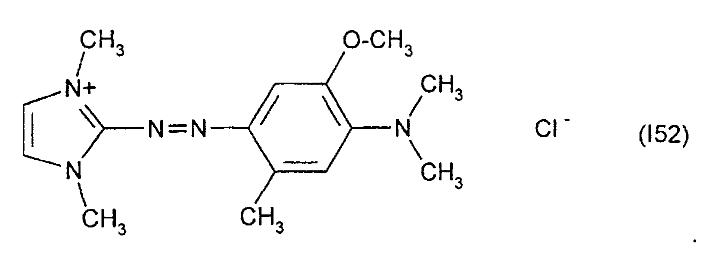

Parmi les composés de structures (I1) à (I52) décrits ci-dessus, on préfère tout particulièrement les composés répondant aux structures (I1), (I2), (I14) et (I31).

Parmi les colorants directs basiques de formule (II), on peut plus particulièrement citer les composés répondant aux structures (II1) à (II12) suivantes : 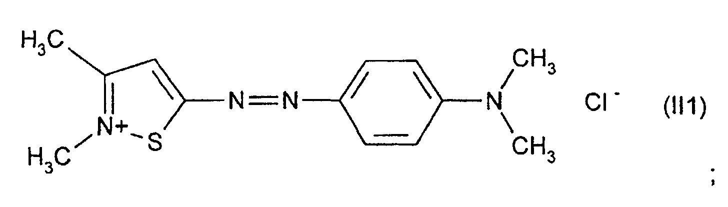 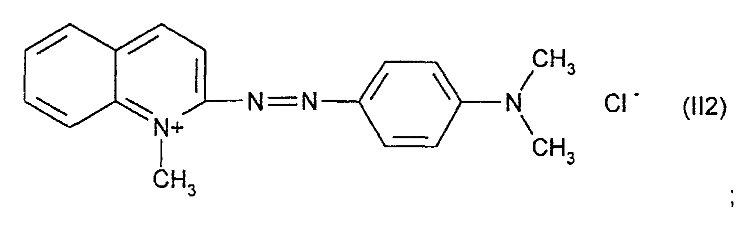 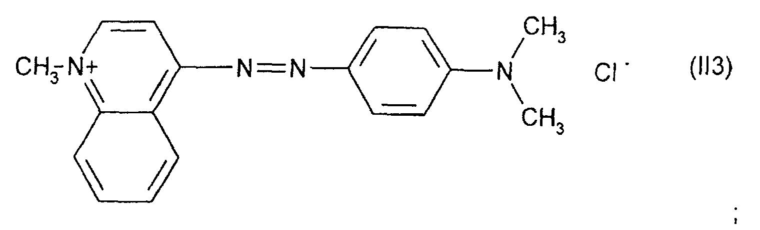 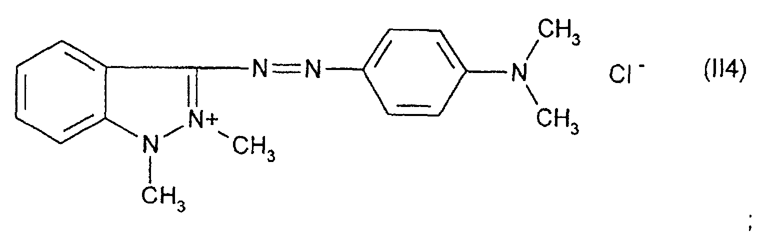 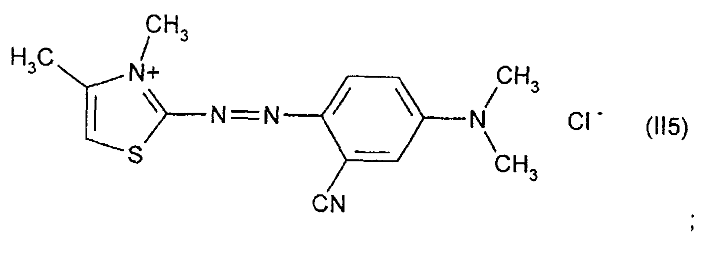 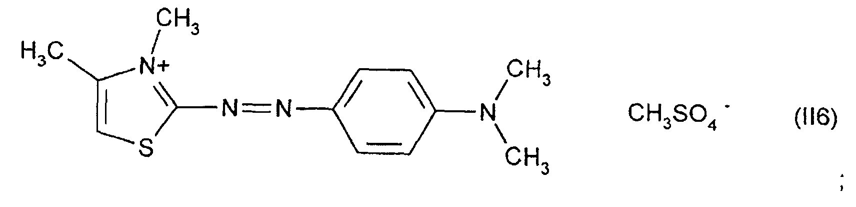 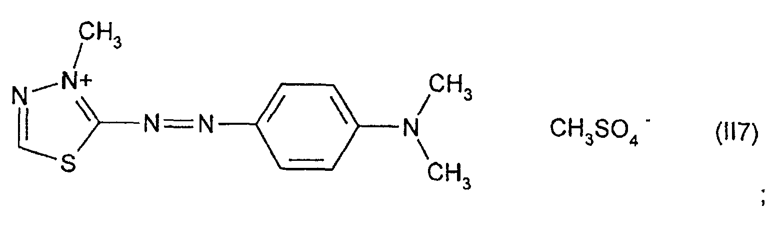 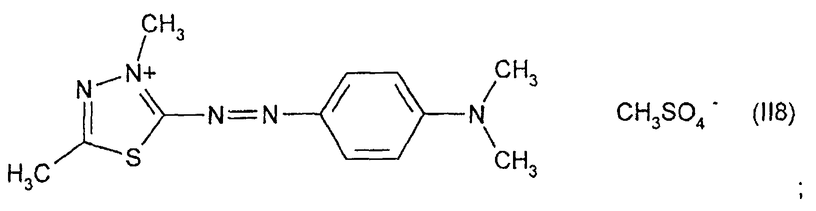 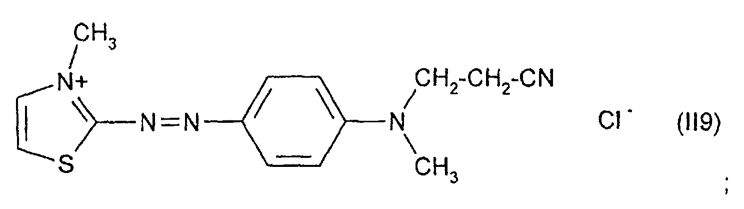 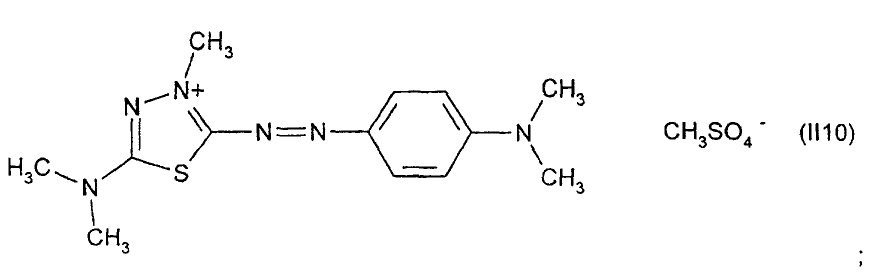 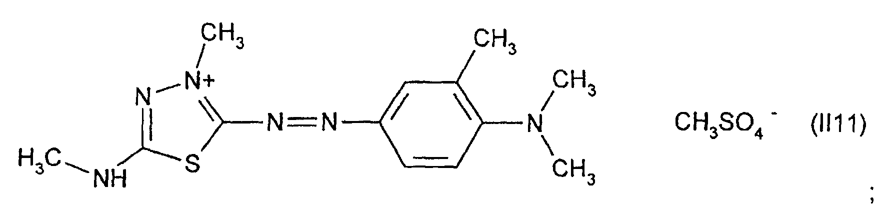 et

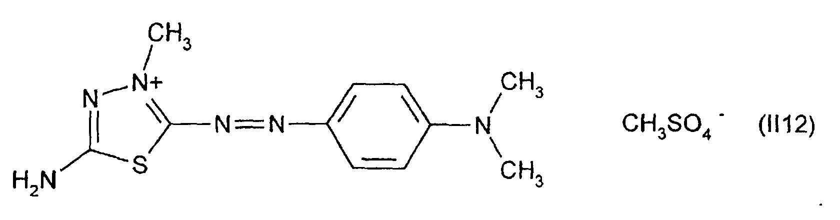

Parmi les colorants directs basiques de formule (III), on peut plus particulièrement citer les composés répondant aux structures (III1) à (III18) suivantes : 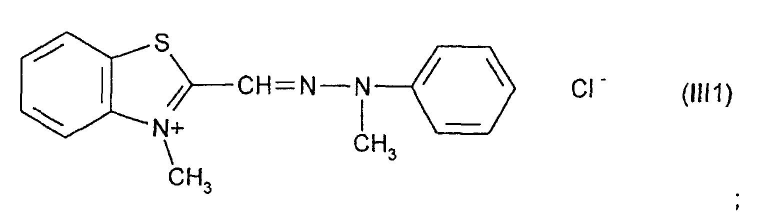 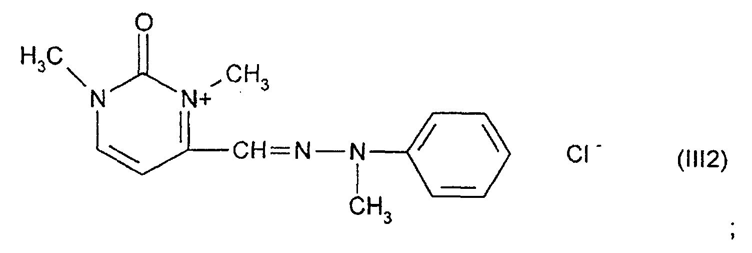 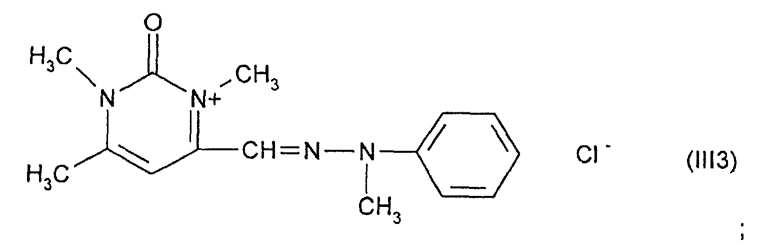 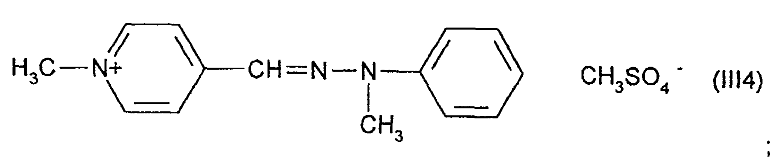 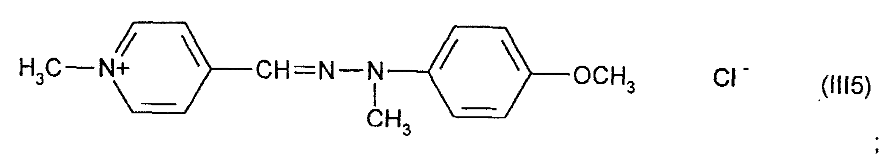 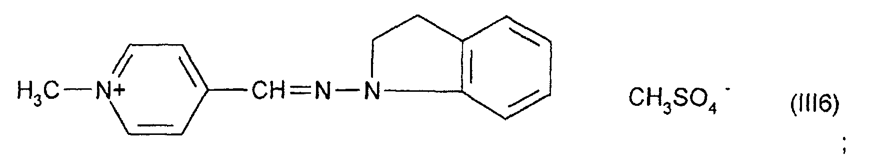 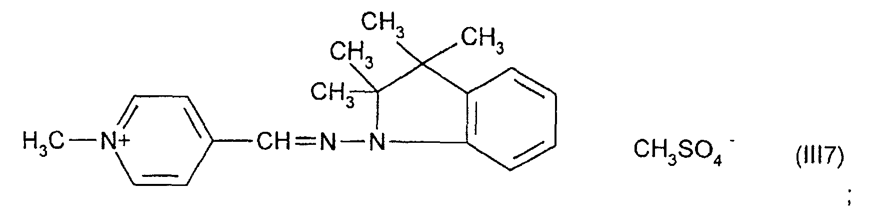 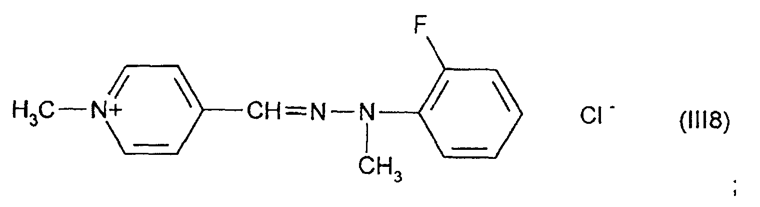 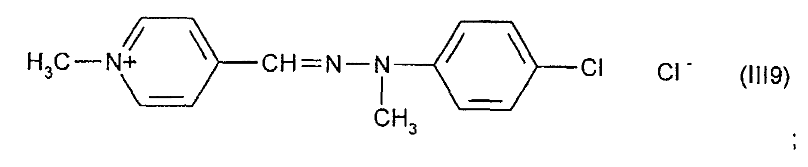 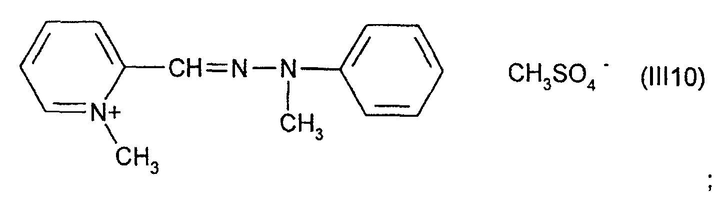 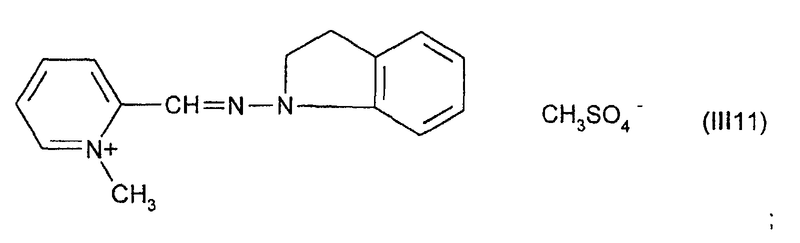 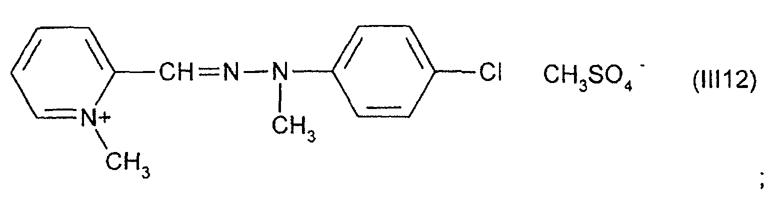 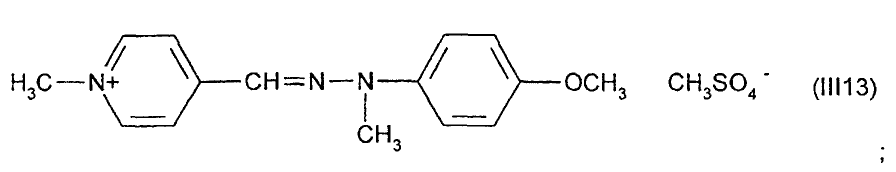 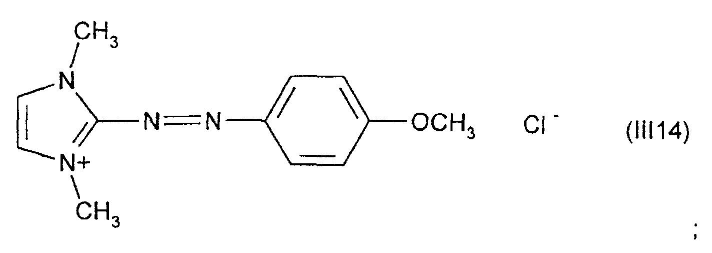 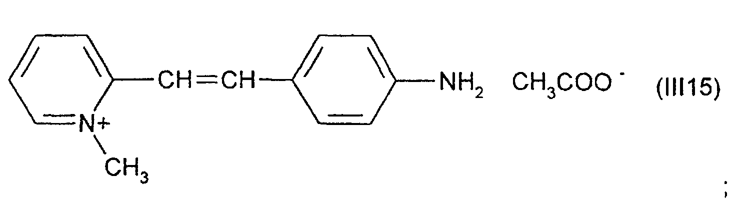 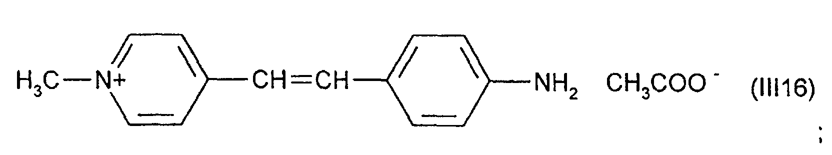 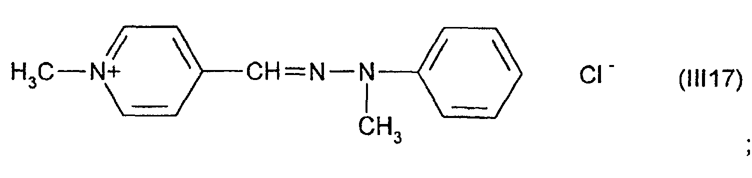 et

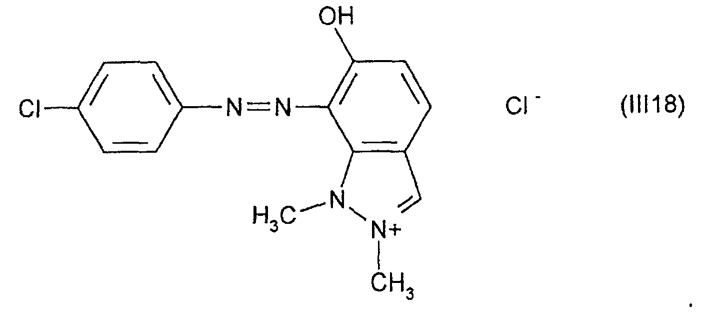

Parmi les composés particuliers de structures (III1) à (III18) décrits ci-dessus, on préfère tout particulièrement les composés répondant aux structures (III4), (III5) et (III13).

Parmi les colorants directs basiques de formule (III'), on peut plus particulièrement citer les composés répondant aux structures (III'1) à (III'3) suivantes : 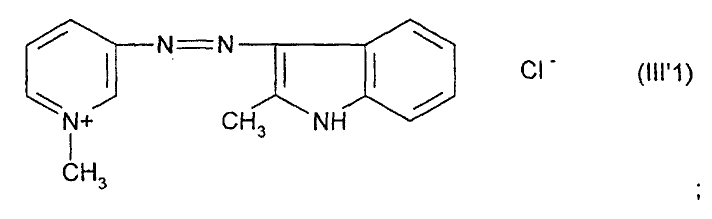 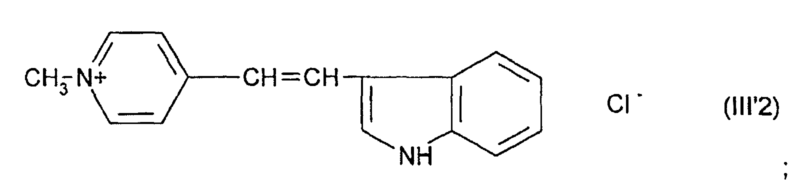 et

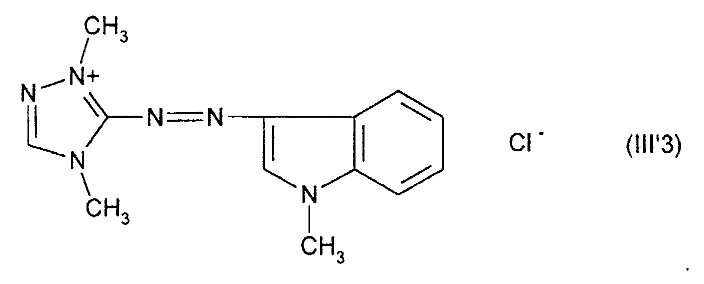

Parmi les colorants directs cationiques de formule (IV) utilisables dans les compositions tinctoriales conformes à l'invention, on peut citer plus particulièrement les composés de structures (IV)₁ à (IV)₇₇ suivantes :

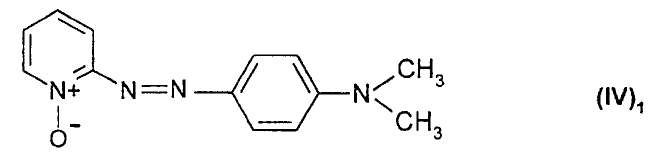

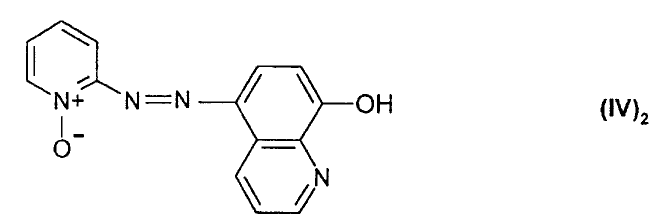

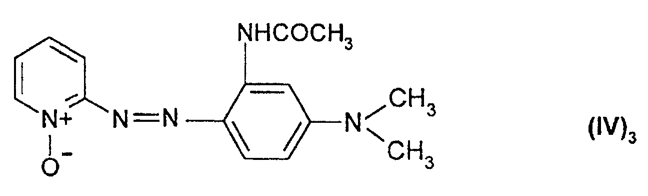

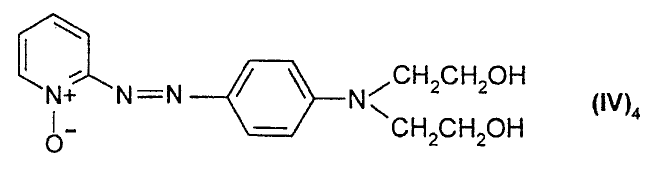

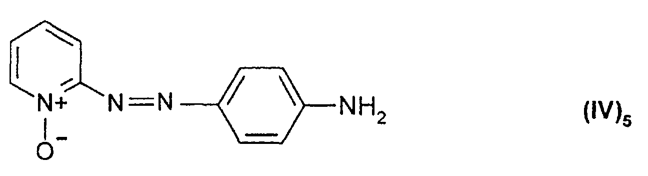

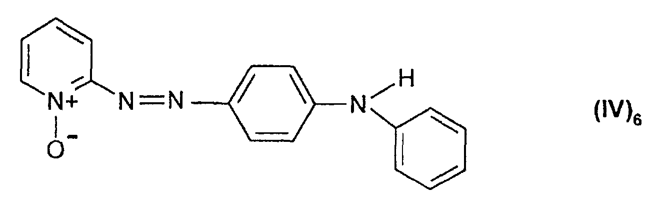

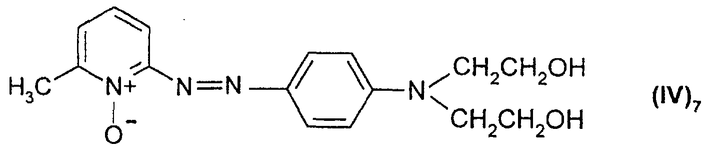

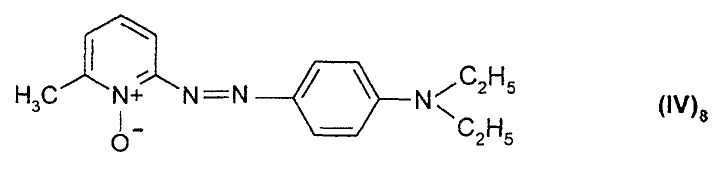

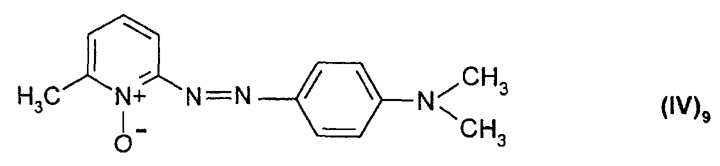

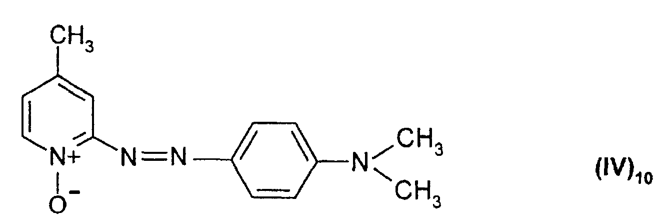

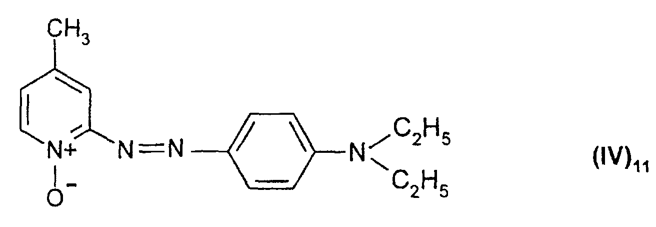

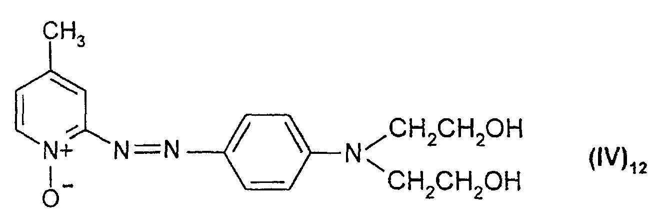

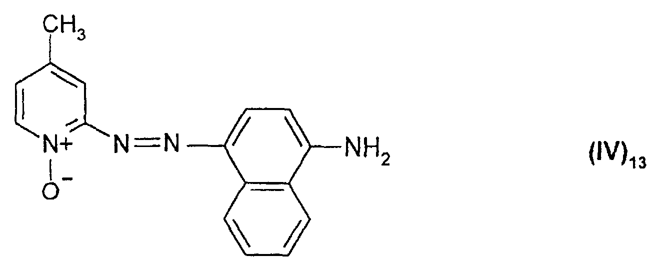

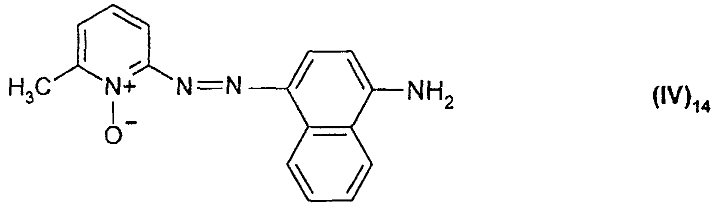

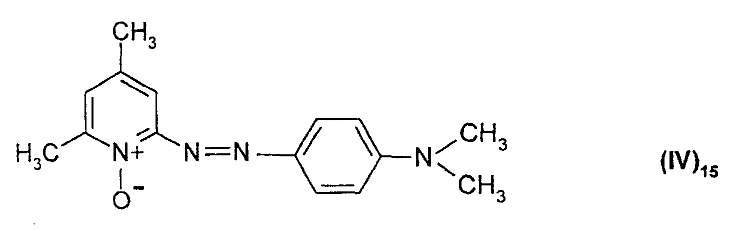

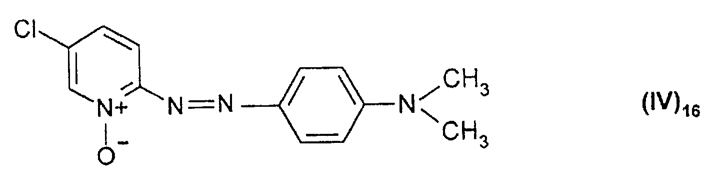

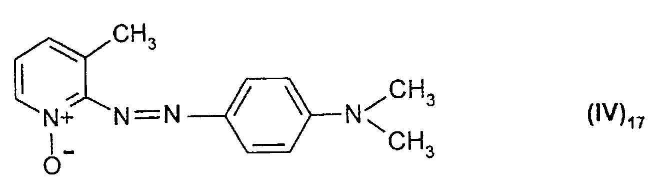

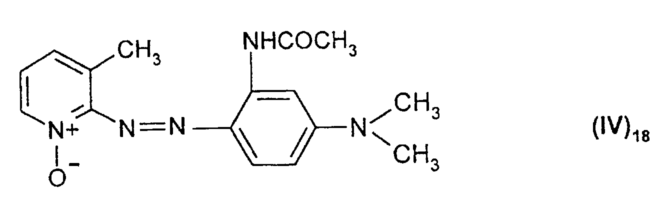

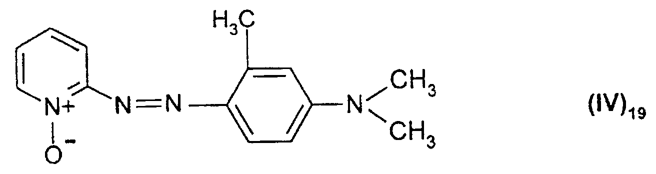

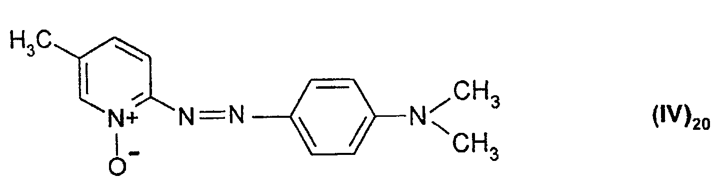

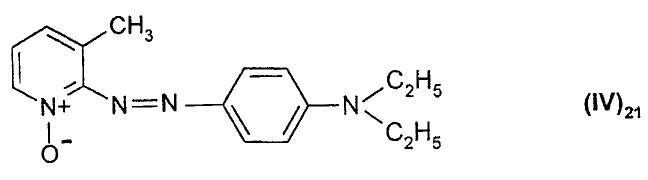

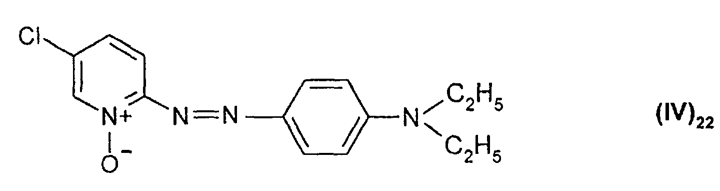

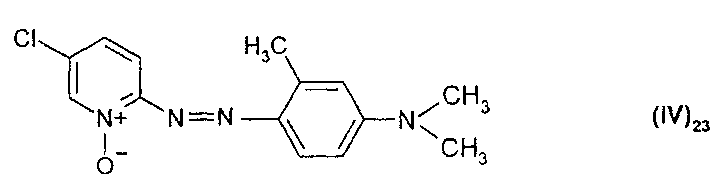

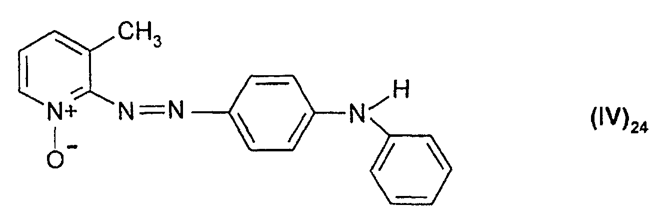

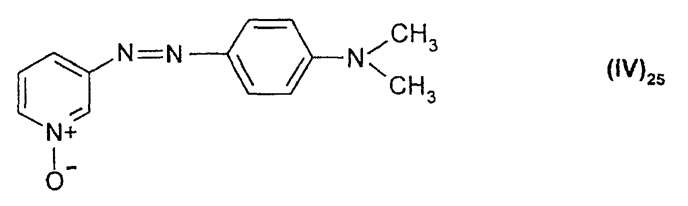

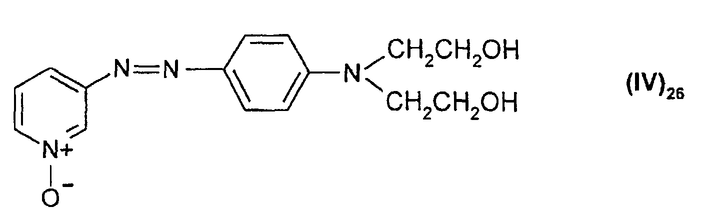

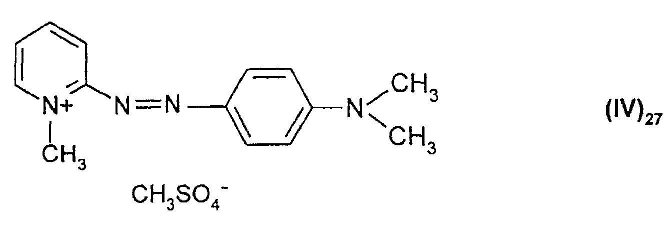

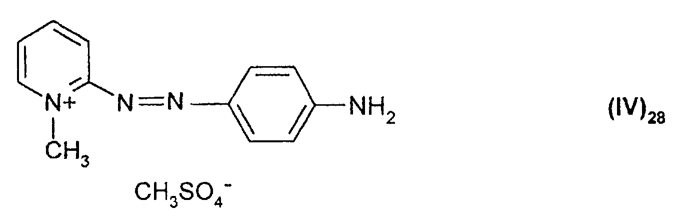

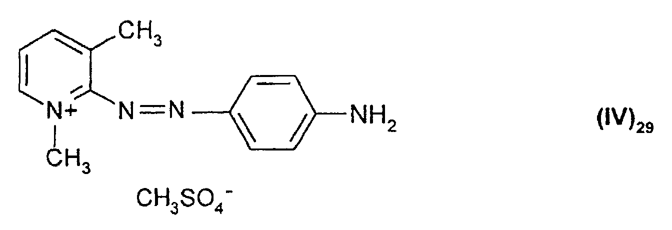

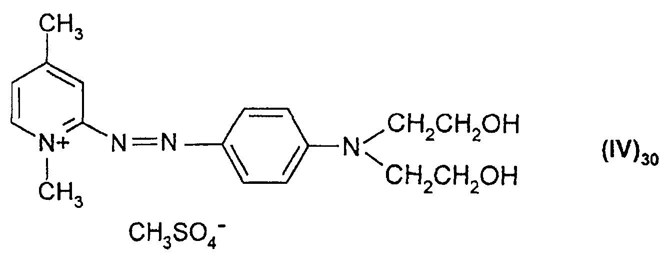

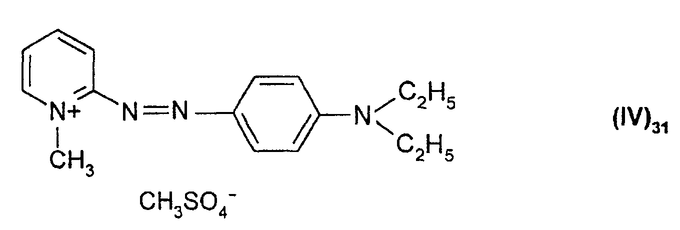

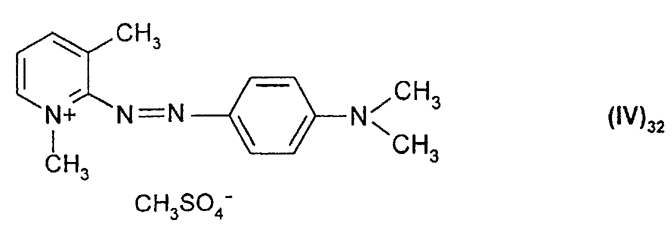

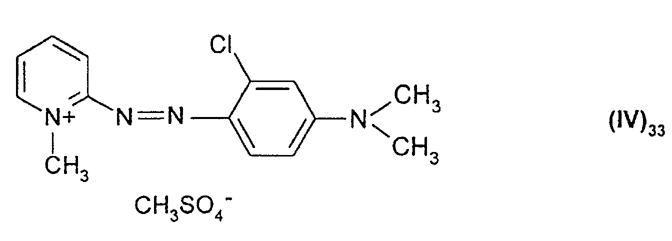

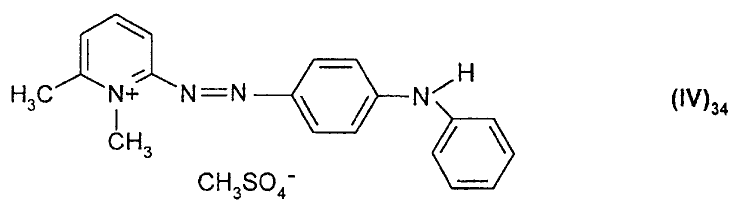

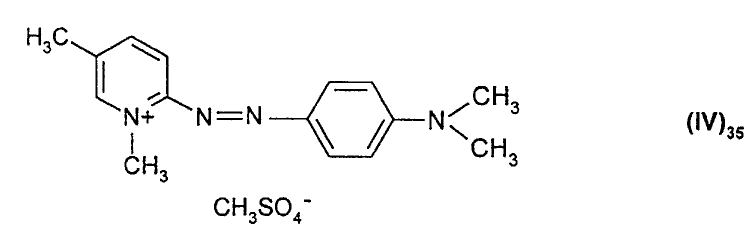

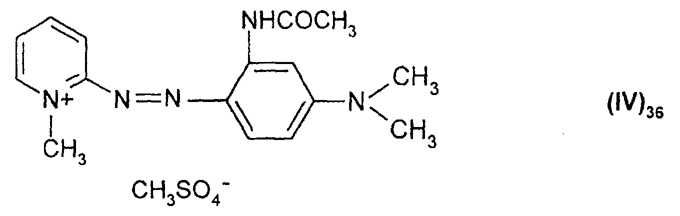

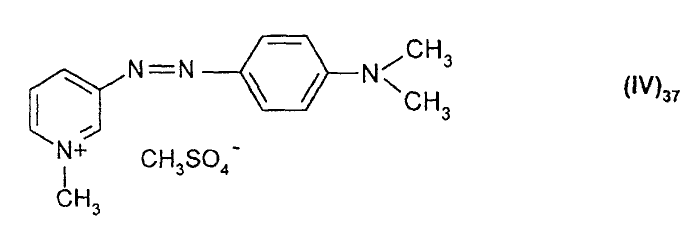

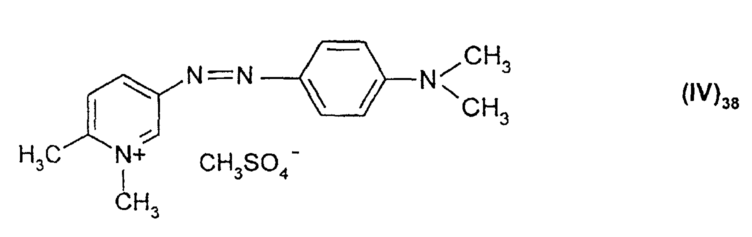

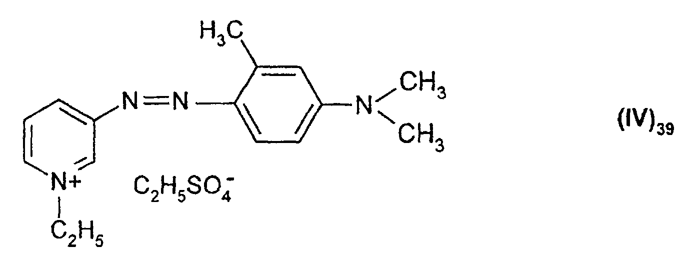

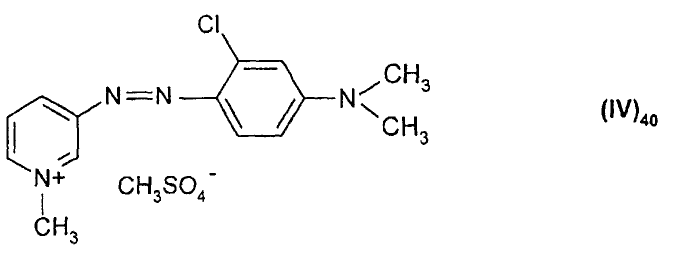

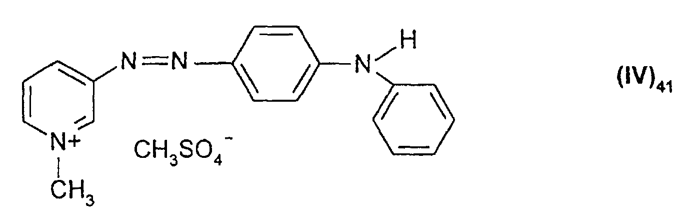

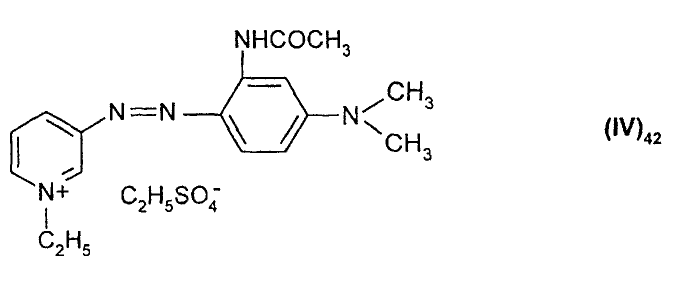

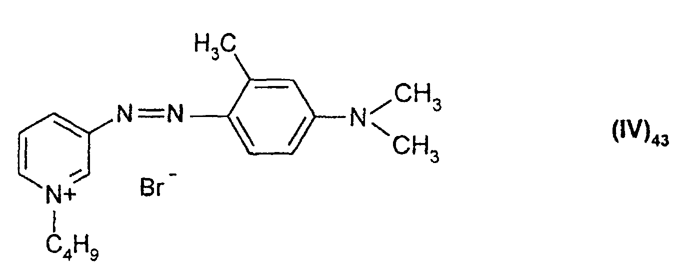

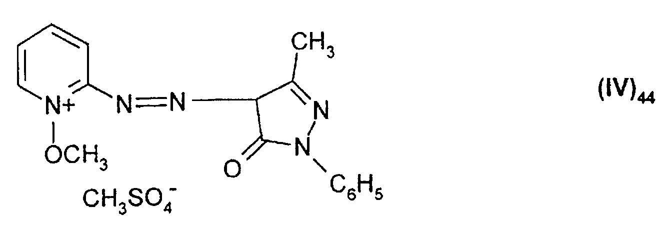

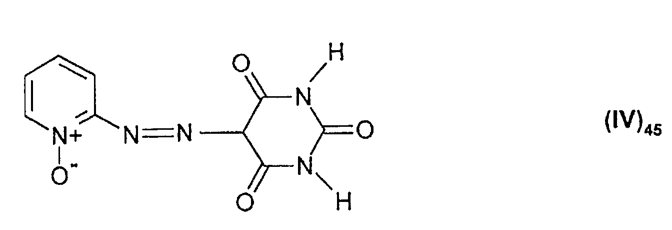

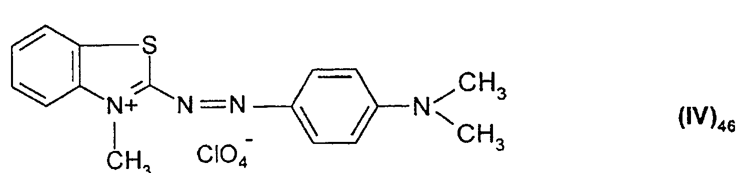

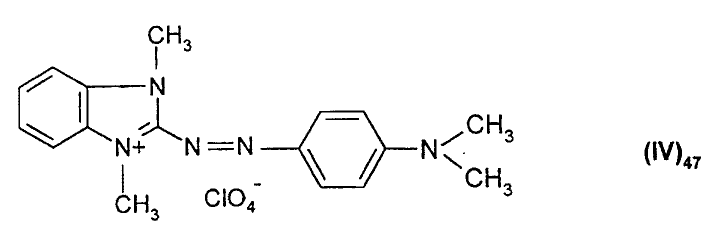

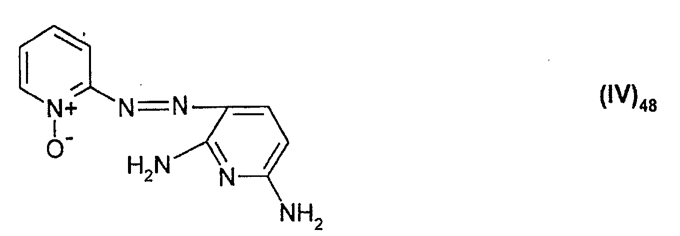

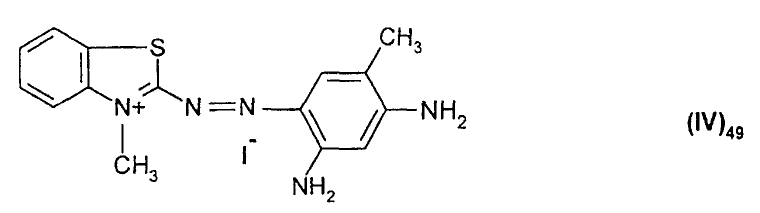

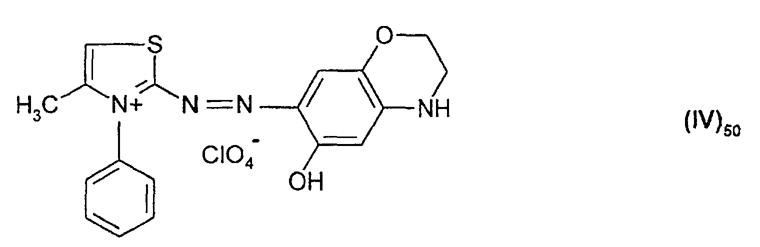

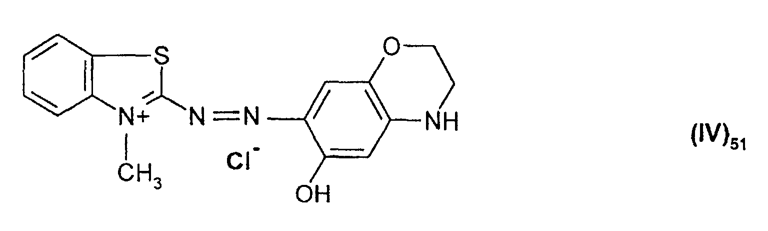

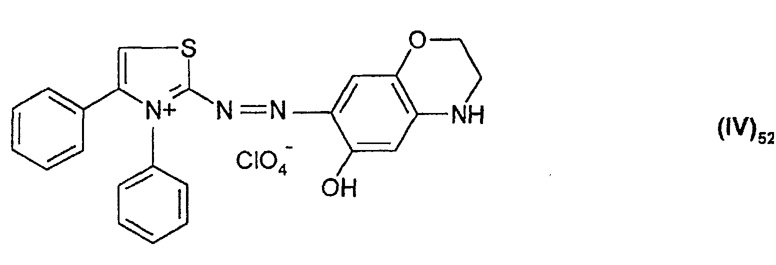

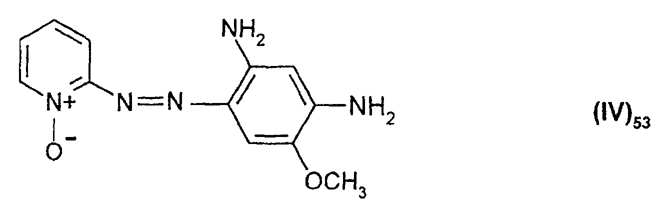

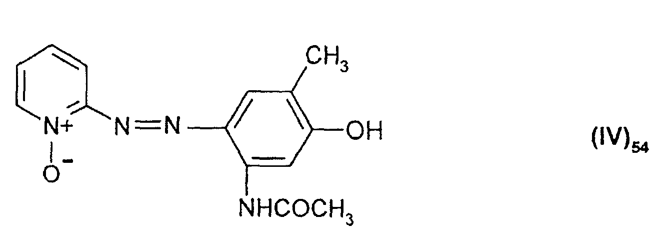

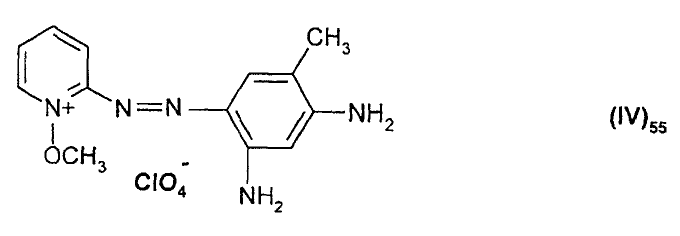

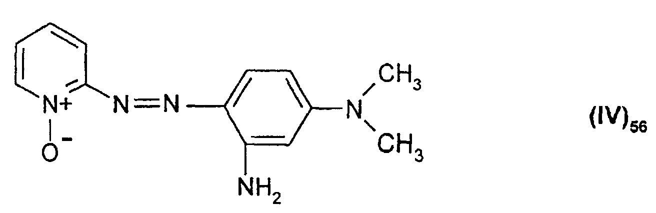

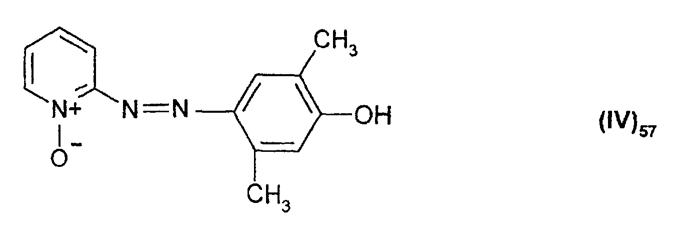

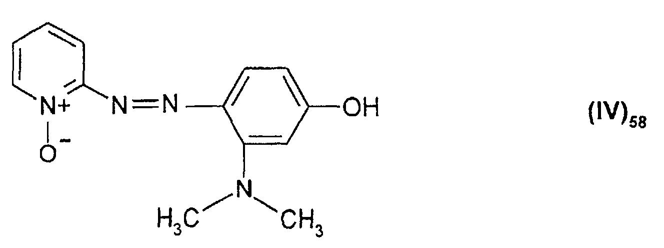

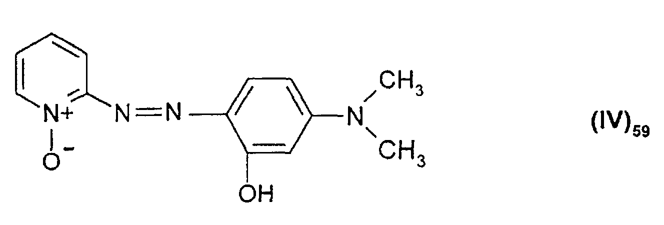

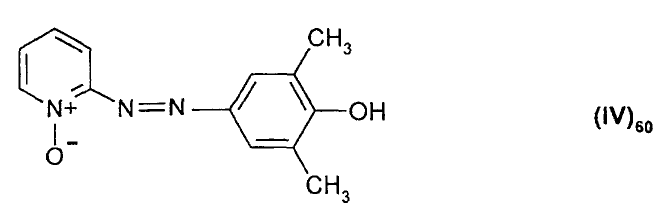

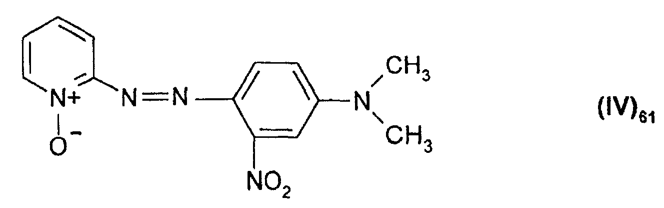

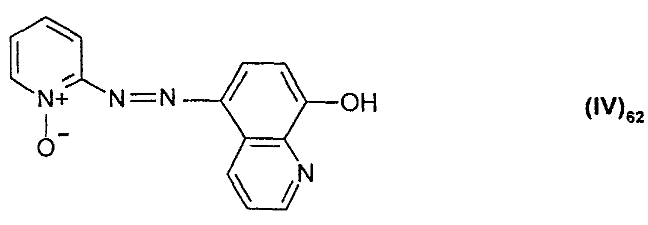

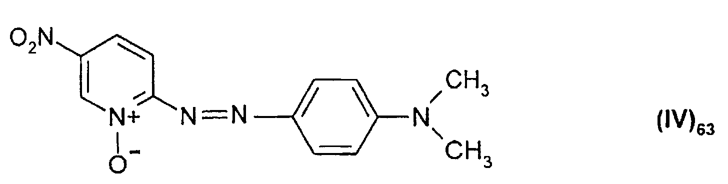

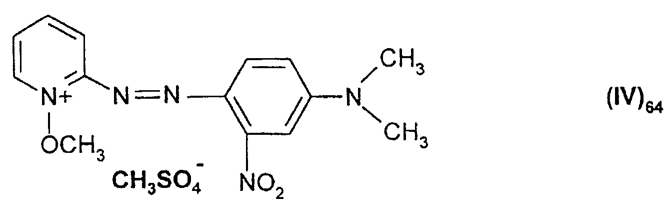

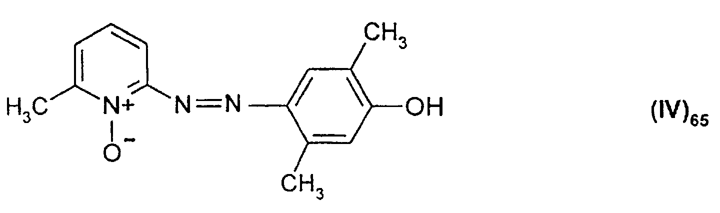

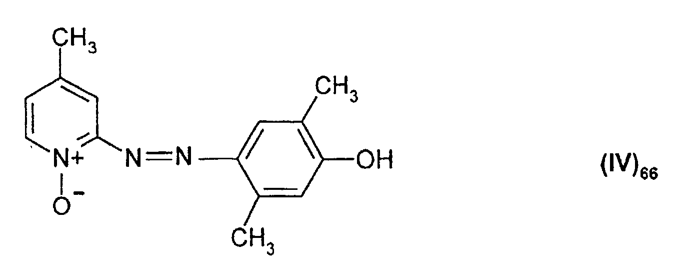

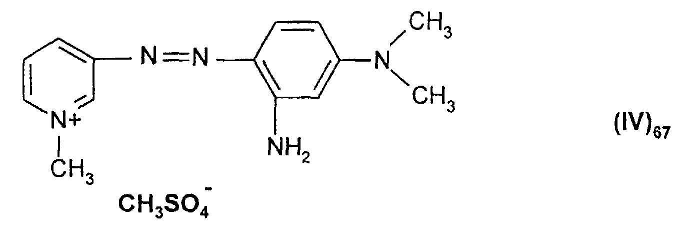

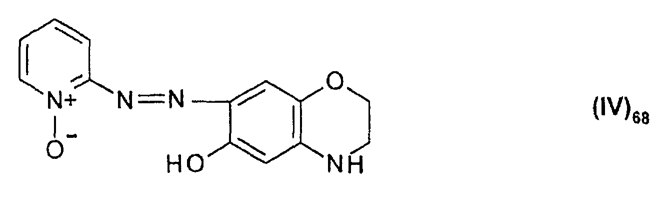

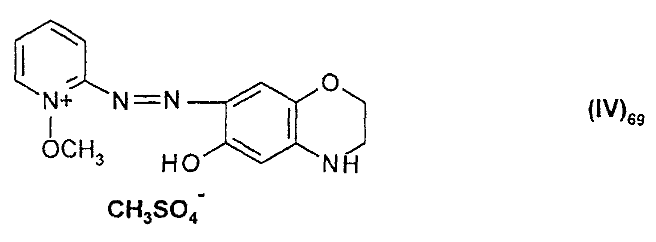

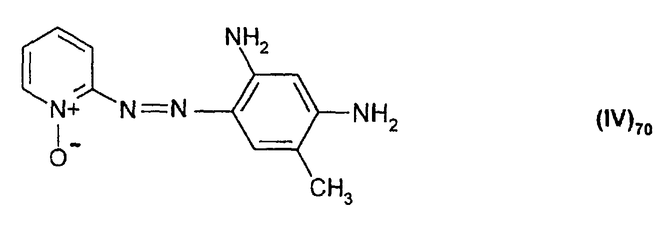

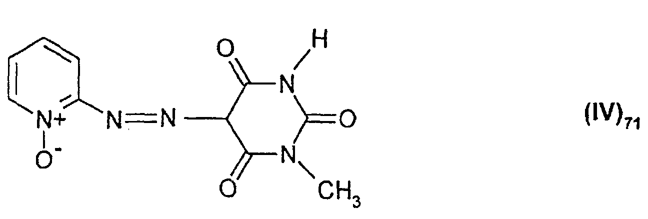

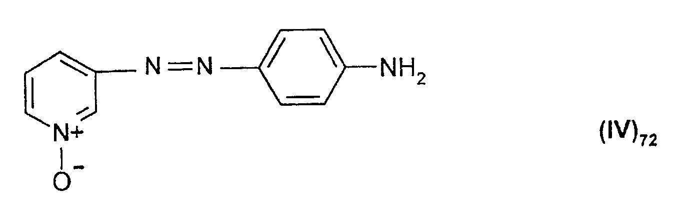

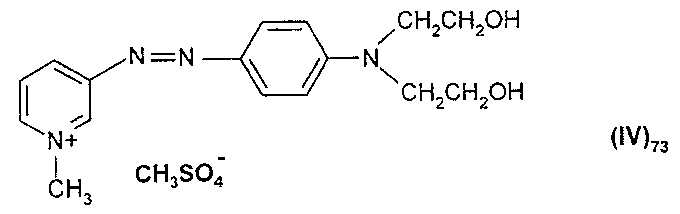

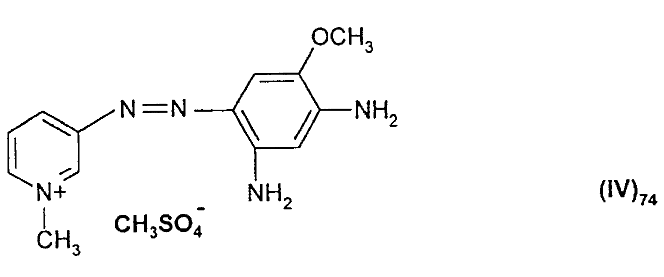

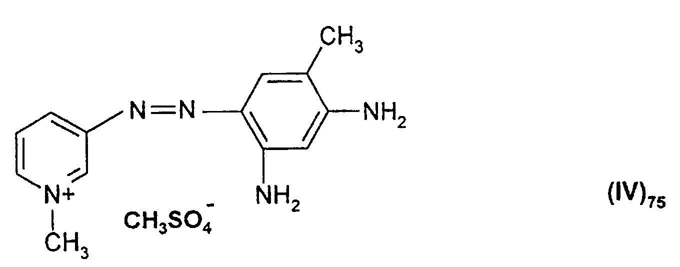

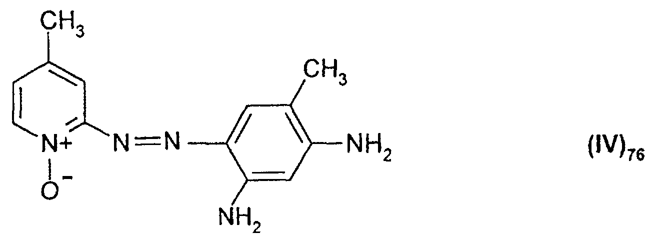

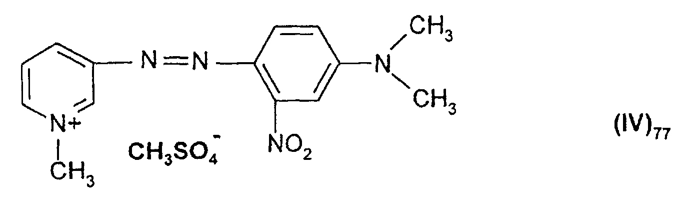

Le milieu aqueux de la composition tinctoriale est constitué uniquement par de l'eau ou par un mélange d'eau et d'au moins un adjuvant cosmétique choisi parmi les divers adjuvants habituellement utilisés pour la teinture des fibres kératiniques tels que des solvants, des agents tensio-actifs, des polymères, des agents épaississants minéraux ou organiques, des agents antioxydants, des agents de pénétration, des agents séquestrants, des parfums, des tampons, des agents dispersants, des agents de conditionnement, des agents filmogènes, des céramides, des agents conservateurs, des agents opacifiants.

Selon l'invention, l'eau représente de préférence de 20 à 95 % en poids du poids total de la composition tinctoriale et encore plus préférentiellement de 40 à 90 % en poids de ce poids.

Selon le procédé de l'invention, la composition tinctoriale peut être prête à l'emploi ou préparée au moment de l'emploi par mélange d'au moins une composition pulvérulente (P) contenant au moins un colorant direct basique et d'au moins une composition aqueuse (A).

La composition aqueuse (A) peut être constituée uniquement d'eau ou d'un mélange d'eau et d'un ou plusieurs adjuvants cosmétiques tels que ceux cités précédemment.

Dans la composition pulvérulente (P), le ou les colorants directs basiques peuvent constituer à eux seuls toute la composition pulvérulente, ou être dispersés au sein d'un excipient, en poudre, de nature organique et/ou de nature minérale. Cette poudre présente de préférence une taille de particules inférieure à 350 µm.

L'excipient organique peut être d'origine synthétique ou végétale et choisi notamment parmi les polymères synthétiques réticulés ou non réticulés, les polysaccharides comme les celluloses et les amidons modifiés ou non ainsi que les produits naturels les renfermant tels que la sciure de bois, ou les gommes végétales (guar, caroube, xanthane, etc...).

L'excipient minéral peut être constitué par des oxydes métalliques tels que les oxydes de titane, les oxydes d'aluminium, le kaolin, le talc, les silicates, le mica et les silices. Un excipient avantageusement préféré est constitué par de la sciure de bois telle que la sciure d'Epicéa.

Les produits insolubles dans l'eau pouvant constituer cet excipient minéral ou organique peuvent également être présents dans la composition tinctoriale à titre d'agent d'adsorption du ou des colorants directs basiques.

La composition pulvérulente (P), peut encore contenir des liants ou produits d'enrobage dans une quantité ne dépassant pas de préférence 3% en poids environ du poids total de ladite composition pulvérulente.

Ces liants sont de préférence des huiles ou corps gras liquides d'origine minérale, synthétique, animale ou végétale.

La composition pulvérulente, peut éventuellement encore contenir d'autres adjuvants, à l'état de poudre, en particulier des tensio-actifs de toute nature, des agents de conditionnement du cheveu.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention, ou à la composition pulvérulente ne soient pas, ou substantiellement pas, altérées par la ou les adjonctions envisagées.

Selon l'invention, la composition tinctoriale utilisée lors de la deuxième étape du procédé est appliquée sur les fibres kératiniques pendant le temps de pause nécessaire à l'obtention de la coloration dans l'intensité désirée qui est compris en général entre 2 et 45 minutes et encore plus particulièrement entre 3 et 10 minutes.

Des exemples concrets illustrant l'invention vont maintenant être donnés, sans pour autant présenter un caractère limitatif.

### EXEMPLES

### EXEMPLES 1 à 3

On a préparé les différentes compositions suivantes :

### Composition oxydante : (commune aux exemples 1, 2 et 3)

Au moment de l'emploi, on a mélangé :
- 48 g d'une composition oxydante en poudre contenant :
   - 70 % en poids d'un mélange de persulfates de sodium et de potassium
   - 12 % en poids de métasilicate de sodium et
   - 7 % de chlorure d'ammonium
- 30 ml d'une crème contenant des tensio-actifs nonioniques et 12 g d'ammoniaque à 20 % de NH₃
- et 30 ml de lait oxydant présentant un pH de 2,0 et titrant 40 volumes (12 % en poids) en peroxyde d'hydrogène.

### Compositions tinctoriales :

Au moment de l'emploi, on a mélangé :
• **60 g d'une composition aqueuse (A) constituée par :**
- Alcool cétylstéarylique 7 g
- Ethanol 2 g
- Diéthanolamide d'acides de coprah étété, vendu sous la dénomination COMPERLAN KD par la société HENKEL 3 g
- Cocoyl amidoéthyl amine N-hydroxyéthyl N-propionate de sodium 6 g M.A.
- Conservateurs et parfums q.s.
- Eau déminéralisée 100 g
* : M.A. Matière Active
• **40 g d'eau, et**
• **3,5 g d'une composition pulvérulente (P) constituée par (teneurs en grammes) :**

| **Exemple** | **1** | **2** | **3** |
|---|---|---|---|
| Chlorhydrate de 1-(γ-aminopropyl)amino anthraquinone (Colorant direct basique) | 13 | - | - |
| Basic Red 76 (Colorant direct basique) | - | 45,7 | - |
| Basic Red 51 (Colorant direct basique) | - | - | 3 |
| Huile de vaseline | 3 | 3 | 3 |
| Sciure d'Epicéa broyée qsp | 100 g | 100 g | 100 g |

La composition oxydante décolorante a été appliquée sur trois mèches de cheveux châtains foncés pendant 40 minutes à température ambiante. Les mèches de cheveux ont été décolorées de 6 tons, en blond très clair.

Après cette première étape de décoloration, les mèches de cheveux ont été rincées, lavées au shampooing, rincées à nouveau puis séchées.

On a ensuite appliqué sur chacune des mèches de cheveux ainsi décolorées, chacune des compositions tinctoriales décrites ci-dessus, pendant 5 minutes.

Après rinçage et séchage, les mèches de cheveux ont été teintes dans les nuances figurant dans le tableau ci-après :

| **EXEMPLE** | **NUANCE OBTENUE** |
|---|---|
| **1** | Fuchsia intense |
| **2** | Rouge cuivré intense |
| **3** | Fuchsia intense |

## Revendications

1. Procédé de teinture directe en deux étapes des fibres kératiniques, et en particulier des fibres kératiniques humaines telles que les cheveux, **caractérisé par le fait que** dans une première étape, on procède à une décoloration des fibres kératiniques, puis dans une deuxième étape, on applique sur les fibres kératiniques une composition tinctoriale contenant, dans un milieu aqueux, au moins un colorant direct basique partiellement dissous, ladite composition tinctoriale étant prête à l'emploi ou résultant du mélange extemporané d'au moins une composition pulvérulente (P) contenant au moins un colorant direct basique et d'une composition aqueuse (A).

2. Procédé selon la revendication 1, **caractérisé par le fait que** le ou les colorants directs basiques sont en sursaturation.

3. Procédé selon la revendication 1, **caractérisé par le fait que** le ou les colorants directs basiques sont partiellement ou totalement adsorbés sur ou enrobés par une charge minérale ou organique insoluble présente dans la composition tinctoriale.

4. Procédé selon l'une quelconque des revendications précédentes, **caractérisé par le fait que** la première étape est une étape de décoloration conduisant à une nuance présentant un hauteur de ton supérieure ou égale à 6.

5. Procédé selon la revendication 4, **caractérisé par** le fait l'étape de décoloration conduit à une nuance présentant une différence de couleur supérieure ou égale à 2 tons par rapport à la couleur des fibres avant la décoloration.

6. Procédé selon l'une quelconque des revendications précédentes, **caractérisé par le fait que** la décoloration est effectuée par application d'une composition oxydante contenant au moins un agent oxydant.

7. Procédé selon la revendication 6, **caractérisé par le fait que** l'agent oxydant est choisi parmi le peroxyde d'hydrogène, le peroxyde d'urée, les bromates de métaux alcalins, les persels, les polythionates, et leurs mélanges.

8. Procédé selon l'une quelconque des revendications 6 ou 7, **caractérisé par le fait que** la composition oxydante se présente sous forme liquide ou crémeuse, ladite composition oxydante étant prête à l'emploi ou résultant du mélange extemporané d'une ou plusieurs compositions aqueuses ou d'une ou plusieurs compositions aqueuses avec une ou plusieurs compositions pulvérulentes, l'agent oxydant étant présent dans la ou les compositions aqueuses et/ou dans la ou les compositions pulvérulentes.

9. Procédé selon l'une quelconque des revendications 6 à 8, **caractérisé par le fait que** la composition oxydante présente un pH compris entre 5 et 12.

10. Procédé selon l'une quelconque des revendications 1 à 5, **caractérisé par le fait que** la décoloration est effectuée par irradiation des fibres kératiniques à l'aide d'un rayonnement laser sous forme d'impulsions de puissance suffisante pour dégrader la mélanine contenue dans les fibres kératiniques.

11. Procédé selon l'une quelconque des revendications précédentes, **caractérisé par le fait que** le ou les colorants directs basiques sont choisis parmi les amino-anthraquinones basiques, les mono- ou di-azoïques basiques, les azométhines basiques, les naphtoquinones basiques, et les colorants basiques à mono-insaturations éthyléniques.

12. Procédé selon la revendication 11, **caractérisé par le fait que** les colorants sont choisis parmi le chlorure de [8-[(p-aminophényl)azol]-7-hydroxy-2-naphtyl]-triméthylammonium (également appelé Basic Brown 16 ou Arianor Mahogany 306002 dans le Color Index), l'association du chlorure de 3-[(4-amino-6-bromo-5,8-dihydro-1-hydroxy-8-imino-5-oxo-2-naphtyl)-amino]-N,N,N-triméthyl-benzénaminium et du chlorure de 3-[(2,6-dibromo-5,8-dihydro-1-hydroxy-8-imino-5-oxo-3-naphtyl)-amino]-N,N,N-triméthyl-benzénaminium (également dénommé Basic Blue 99 ou Arianor Steel Blue 306004 dans le Color Index), le chlorure de 7-hydroxy-8-[(2-méthoxyphényl)azo]-N,N,N-triméthyl-2-naphtalènaminium (également appelé le Basic Red 76 ou Arianor Madder Red dans le Color Index), le chlorure de [8-[(4-amino-2-nitrophényl)azo]-7-hydroxy-2-naphtyl]-triméthylammonium (également appelé Basic Red 118 dans le Color Index), l'association du chlorure de [8-[(4-amino-3-nitrophényl)azo]-7-hydroxy-2-naphtyl]-triméthylammonium et du chlorure de [8-[(4-amino-2-nitrophényl)azo]-7-hydroxy-2-naphtyl]-triméthylammonium (également appelé Brown 17 ou Arianor Sienna Brown 306001 dans le Color Index), le chlorure de 3-[(4,5-dihydro-3-méthyl-5-oxo-1-phényl-1H-pyrazol-4-yl)azo]-N,N,N-triméthyl-benzènaminium (également appelé Basic Yellow 57 ou Arianor Straw Yellow 306005 dans le Color Index), le chlorhydrate de 1-(γ-aminopropyl)amino anthraquinone, le méthylsulfate de 1-N-(méthyl morpholinium propyl)amino 4-hydroxy anthraquinone, et le Basic Orange 69 (dénomination du Color Index).

13. Procédé selon l'une quelconque des revendications 1 à 10, **caractérisé par le fait que** le ou les colorants directs basiques sont choisis parmi :
**a) les composés de formule (I) suivante :** 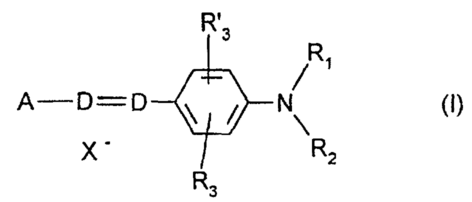 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄; un radical 4'-aminophényle,
R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
X ⁻ représente un anion de préférence choisi parmi le chlorure, le méthyl sulfate et l'acétate,
A représente un groupement choisi par les structures A1 à A19 suivantes : 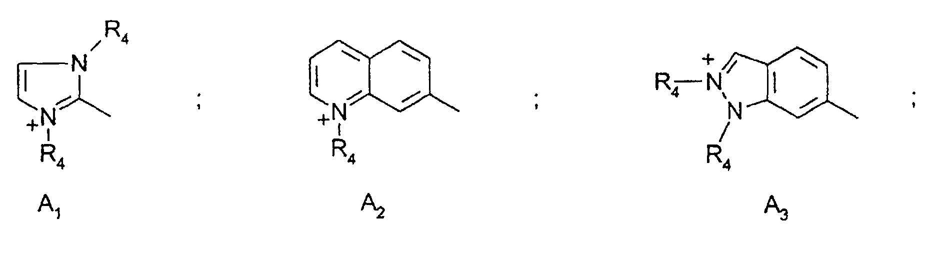 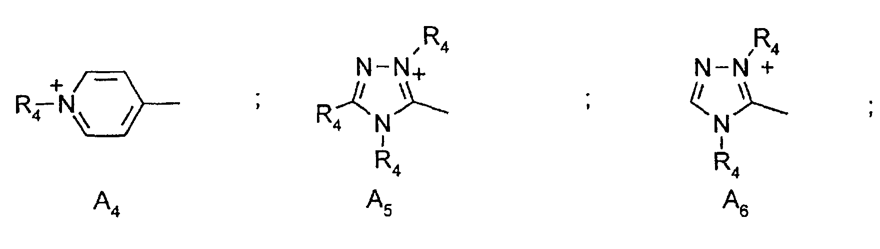 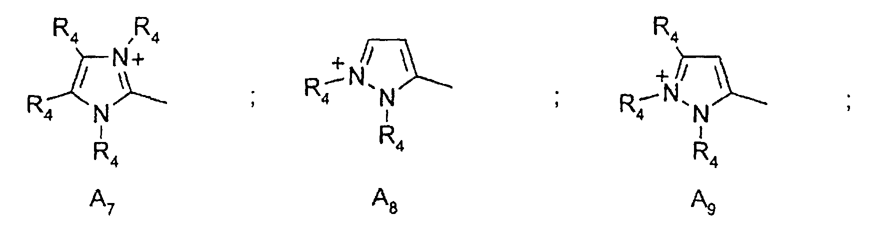 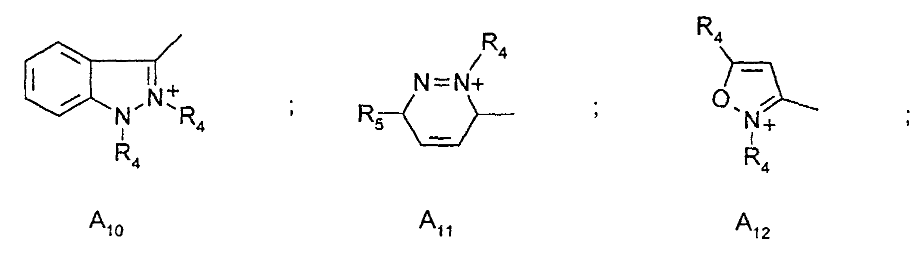 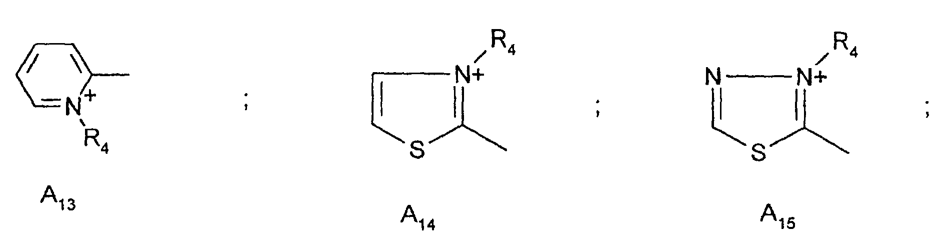 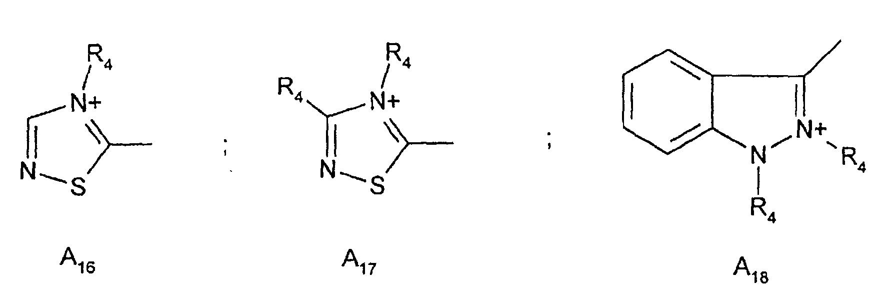 et 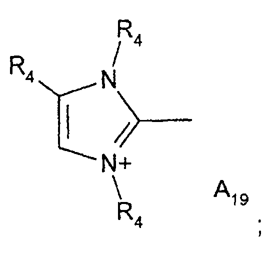
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 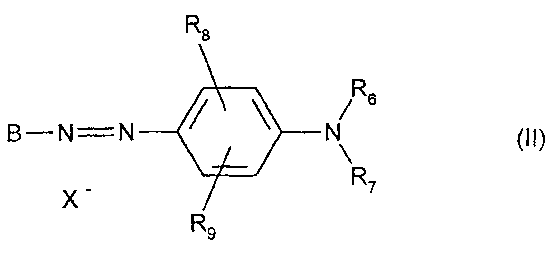 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X ⁻ représente un anion de préférence choisi parmi le chlorure, le méthyl sulfate et l'acétate,
B représente un groupement choisi par les structures B1 à B6 suivantes : 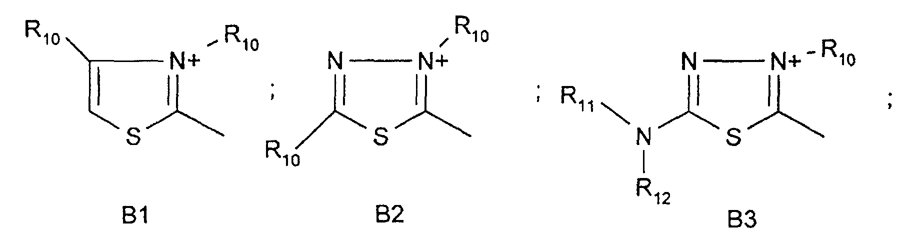 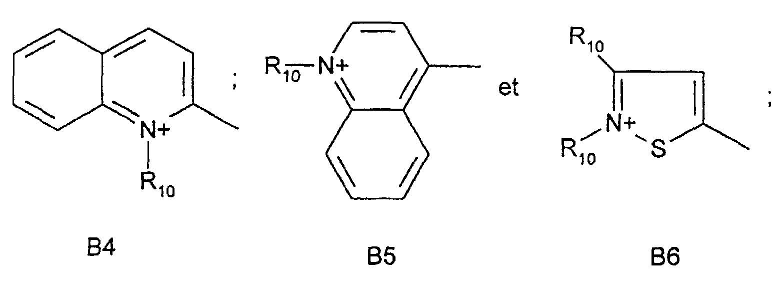 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 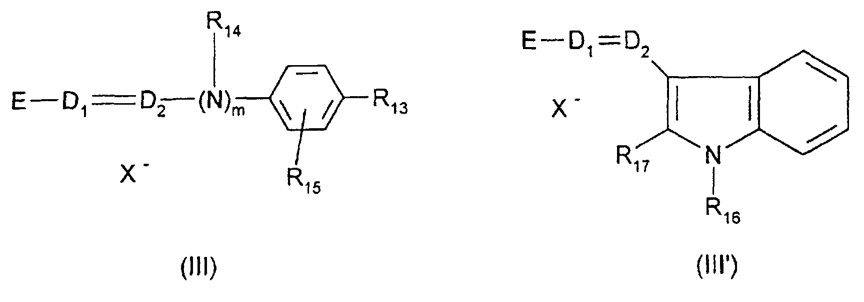 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X ⁻ représente un anion de préférence choisi parmi le chlorure, le méthyl sulfate et l'acétate,
E représente un groupement choisi par les structures E1 à E8 suivantes : 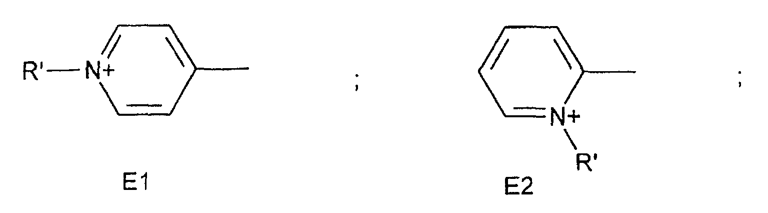 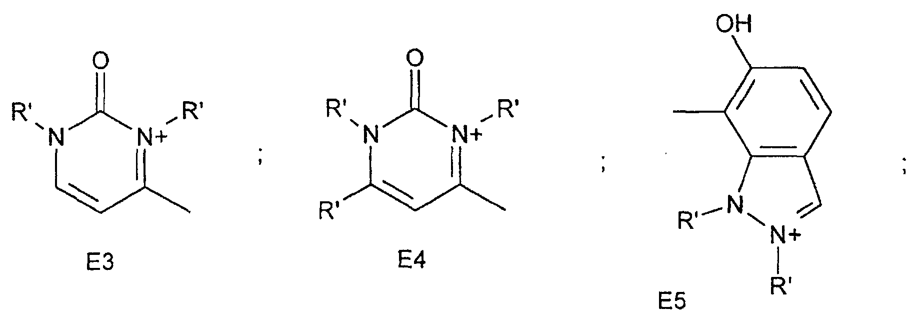 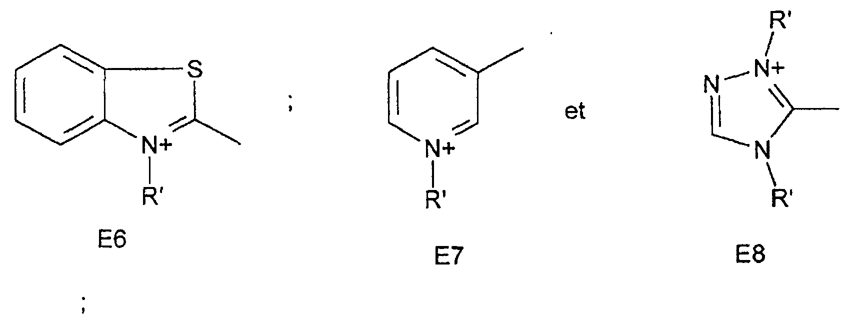 dans lesquelles R' représente un radical alkyle en C₁-C₄ ;
lorsque m = 0 et que D₁ représente un atome d'azote, alors E peut également désigner un groupement de structure E9 suivante : 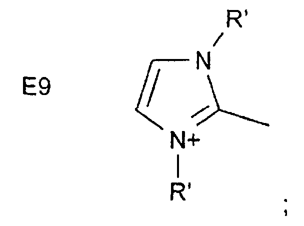 dans laquelle R' représente un radical alkyle en C₁-C₄;
**d) les composés de formule (IV) suivante :**
**G―N ̿N―J** **(IV)**
dans laquelle :
**le symbole G** représente un groupement choisi parmi les structures G₁ à G₃ suivantes : 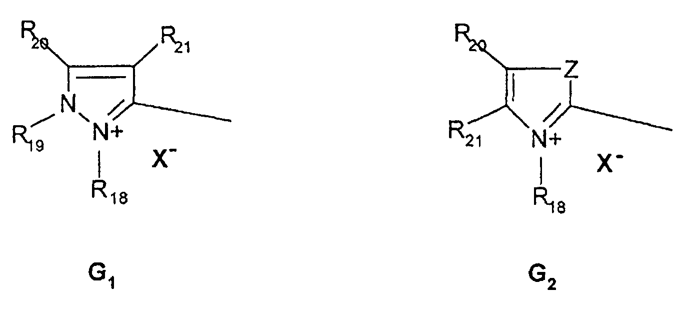 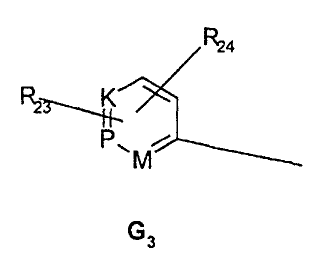 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄ , un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄), ou -NR₂₂(X⁻)ᵣ;
K représente un groupement -CH, -CR (R désignant alkyle en C₁-C₄), ou -NR₂₂(X⁻)ᵣ;
P représente un groupement -CH, -CR (R désignant alkyle en C₁-C₄), ou -NR₂₂(X⁻)ᵣ; r désigne zéro ou 1;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻, alors R₂₃ ou R₂₄ est différent d'un atome d'hydrogène;
si K désigne -NR₂₂(X⁻)ᵣ, alors M= P= -CH, -CR;
si M désigne -NR₂₂(X⁻)ᵣ, alors K= P= -CH, -CR;
si P désigne -NR₂₂(X⁻)ᵣ, alors K= M et désignent -CH ou -CR;
si Z désigne un atome de soufre avec R₂₁ désignant alkyle en C₁-C₄ , alors R₂₀ est différent d'un atome d'hydrogène;
si Z désigne -NR₂₂ avec R₁₉ désignant alkyle en C₁-C₄ , alors au moins l'un des radicaux R₁₈, R₂₀ ou R₂₁ de G₂ est différent d'un radical alkyle en C₁-C₄;
**le symbole J** représente :
- **(a)** un groupement de structure J₁ suivante : 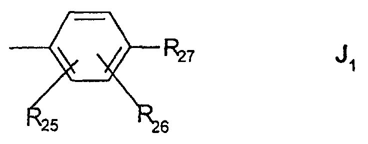 structure J₁ dans laquelle,
R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
R₂₇ représente un atome d'hydrogène, un radical -OH, un radical -NHR₂₈, un radical -NR₂₉R₃₀;
R₂₈ représente un atome d'hydrogène, un radical alkyle en C₁-C₄, un radical monohydroxyalkyle en C₁-C₄, polyhydroxyalkyle en C₂-C₄, un radical phényle;
R₂₉ et R₃₀, identiques ou différents, représentent un radical alkyle en C₁-C₄ , un radical monohydroxyalkyle en C₁-C₄, polyhydroxyalkyle en C₂-C₄;
- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 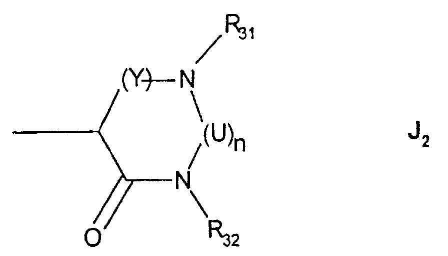 structure J₂ dans laquelle,
R₃₁ et R₃₂, identiques ou différents, représentent un atome d'hydrogène , un radical alkyle en C₁-C₄, un radical phényle;
Y désigne le radical -CO- ou le radical 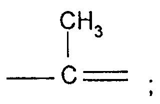 n = 0 ou 1, avec, lorsque n désigne 1, U désigne le radical -CO- .

14. Procédé selon l'une quelconque des revendications précédentes, **caractérisé par le fait que** le milieu aqueux de la composition tinctoriale est constitué uniquement par de l'eau ou par un mélange d'eau et d'au moins un adjuvant cosmétique choisi parmi les solvants, les agents tensio-actifs, les polymères, les agents épaississants minéraux ou organiques, les agents antioxydants, les agents de pénétration, les agents séquestrants, les parfums, les tampons, les agents dispersants, les agents de conditionnement, les agents filmogènes, les céramides, les agents conservateurs et les agents opacifiants.

15. Procédé selon l'une quelconque des revendications précédentes, **caractérisé par le fait que** l'eau représente de 20 à 95 % en poids du poids total de la composition tinctoriale.

16. Procédé selon l'une quelconque des revendications précédentes, **caractérisé par le fait que** dans la composition pulvérulente (P), le ou les colorants directs basiques constituent à eux seuls toute la composition pulvérulente, ou sont dispersés au sein d'un excipient, en poudre, de nature organique et/ou de nature minérale.

17. Procédé selon la revendication 16, **caractérisé par le fait que** l'excipient est la sciure de bois.

18. Procédé selon l'une quelconque des revendications précédentes, **caractérisé par le fait que** la composition tinctoriale est appliquée pendant un temps de pause compris entre 2 et 45 minutes.

19. Procédé selon la revendication 18, **caractérisé par le fait que** le temps de pause est compris entre 3 et 10 minutes.

## Patentansprüche

1. Zweistufiges Verfahren zum direkten Färben von Keratinfasern und insbesondere menschlichen Keratinfasern, wie dem Haar, **dadurch gekennzeichnet, dass** in einem ersten Schritt die Keratinfasern entfärbt werden und anschließend in einem zweiten Schritt auf die Keratinfasern eine Farbmittelzusammensetzung aufgetragen wird, die in einem wässrigen Medium mindestens einen teilweise gelösten, basischen Direktfarbstoff enthält, wobei die Farbmittelzusammensetzung gebrauchsfertig ist oder durch Mischen mindestens einer pulverförmigen Zusammensetzung (P), die mindestens einen basischen Direktfarbstoff enthält, und einer wässrigen Zusammensetzung (A) unmittelbar vor der Anwendung gebildet wird.

2. Verfahren nach Anspruch 1, **dadurch gekennzeichnet, dass** der oder die basischen Direktfarbstoffe in Übersättigung vorliegen.

3. Verfahren nach Anspruch 1, **dadurch gekennzeichnet, dass** der oder die basischen Direktfarbstoffe ganz oder teilweise an einem in der Farbmittelzusammensetzung vorliegenden, unlöslichen, anorganischen oder organischen Füllstoff adsorbiert sind oder von einem solchen Füllstoff umhüllt sind.

4. Verfahren nach einem der vorhergehenden Ansprüche, **dadurch gekennzeichnet, dass** der erste Schritt eine Entfärbung ist, die zu einer Farbnuance mit einem Farbton von mindestens 6 führt.

5. Verfahren nach Anspruch 4, **dadurch gekennzeichnet, dass** die Entfärbung zu einer Farbnuance führt, die im Vergleich mit der Farbe der Fasern vor der Entfärbung eine Farbdifferenz von mindestens 2 Farbtönen aufweist.

6. Verfahren nach einem der vorhergehenden Ansprüche, **dadurch gekennzeichnet, dass** das Entfärben durch Aufbringen einer oxidierenden Zusammensetzung, die mindestens ein Oxidationsmittel enthält, erfolgt.

7. Verfahren nach Anspruch 6, **dadurch gekennzeichnet, dass** das Oxidationsmittel unter Wasserstoffperoxid, Harnstoffperoxid, Alkalimetallbromaten, Salzen von Persäuren, Polythionaten und deren Gemischen ausgewählt ist.

8. Verfahren nach einem der Ansprüche 6 oder 7, **dadurch gekennzeichnet, dass** die oxidierende Zusammensetzung in flüssiger Form oder als Creme vorliegt, wobei die oxidierende Zusammensetzung gebrauchsfertig ist oder durch Mischen einer oder mehrerer wässriger Zusammensetzungen oder einer oder mehrerer wässriger Zusammensetzungen mit einer oder mehreren pulverförmigen Zusammensetzungen kurz vor der Anwendung hergestellt wird, wobei das Oxidationsmittel in der oder den wässrigen Zusammensetzungen und/oder in der oder den pulverförmigen Zusammensetzungen vorliegt.

9. Verfahren nach einem der Ansprüche 6 bis 8, **dadurch gekennzeichnet, dass** die oxidierende Zusammensetzung einen pH-Wert im Bereich von 5 bis 12 aufweist.

10. Verfahren nach einem der Ansprüche 1 bis 5, **dadurch gekennzeichnet, dass** das Entfärben durch Bestrahlen der Keratinfasern mit einem Laserstrahl in Form von Pulsen mit einer Leistung erfolgt, die ausreichend ist, um das in den Keratinfasern enthaltene Melanin zu zersetzen.

11. Verfahren nach einem der vorhergehenden Ansprüche, **dadurch gekennzeichnet, dass** der oder die basischen Direktfarbstoffe unter den basischen Aminoanthrachinonen, basischen Monooder Diazo-Farbstoffen, basischen Azomethinen, basischen Naphthochinonen und den monoethylenisch ungesättigten basischen Farbstoffen ausgewählt sind.

12. Verfahren nach Anspruch 11, **dadurch gekennzeichnet, dass** die Farbstoffe unter [8-[(p-Aminophenyl)azo]-7-hydroxy-2-naphthyl]-trimethylammoniumchlorid (das nach dem Color Index auch als Basic Brown 16 oder Arianor Mahogany 306002 bezeichnet wird), der Kombination von 3-[(4-Amino-6-brom-5,8-dihydro-1-hydroxy-8-imino-5-oxo-2-naphthyl)-amino]-N,N,N-trimethylbenzolaminiumchlorid und 3-[(2,6-Dibrom-5,8-dihydro-1-hydroxy-8-imino-5-oxo-3-naphthyl)-amino]-N,N,N-trimethylbenzolaminiumchlorid (nach dem Color Index auch als Basic Blue 99 oder Arianor Steel Blue 306004 bezeichnet), 7-Hydroxy-8-[(2-methoxyphenyl)azo]-N,N,N-trimethyl-2-naphthalinaminiumchlorid (auch Basic Red 76 oder Arianor Madder Red nach dem Color Index), [8-[(4-Amino-2-nitrophenyl)azo]-7-hydroxy-2-naphthyl]trimethylammoniumchlorid (nach dem Color Index auch Basic Red 118), der Kombination von [8-[(4-Amino-3-nitrophenyl)azo]-7-hydroxy-2-naphthyl]-trimethylammoniumchlorid und [8-[(4-Amino-2-nitrophenyl)azo]-7-hydroxy-2-naphthyl]-trimethylammoniumchlorid (auch Brown 17 oder Arianor Sienna Brown 306001 nach dem Color Index), 3-[(4,5-Dihydro-3-methyl-5-oxo-1-phenyl-1H-pyrazol-4-yl)azo]-N,N,N-trimethyl-benzolaminiumchlorid (Basic Yellow 57 oder Arianor Straw Yellow 306005 nach dem Color Index), 1-(γ-Aminopropyl)aminoanthrachinon-Hydrochlorid, 1-N-(Methylmorpholiniumpropyl)amino-4-hydroxyanthrachinon-methylsulfat und Basic Orange 69 (Color Index-Bezeichnung) ausgewählt sind.

13. Verfahren nach einem der Ansprüche 1 bis 10, **dadurch gekennzeichnet, dass** der oder die basischen Direktfarbstoffe unter den folgenden Verbindungen ausgewählt sind:
**a) Verbindungen der folgenden Formel (I):** 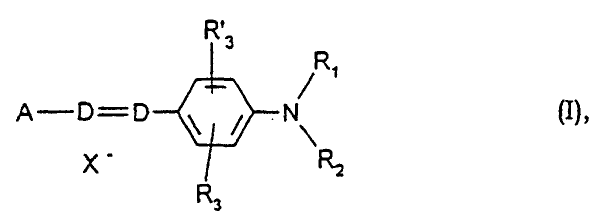 worin bedeuten:
D ein Stickstoffatom oder die Gruppe -CH, die Gruppen R₁ und R₂, die gleich oder verschieden sind, ein Wasserstoffatom; eine C₁₋₄-Alkylgruppe, die mit einer Gruppe - CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder eine 4'-Aminophenylgruppe,
die Gruppen R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkoxy oder Acetyloxy,
X- ein Anion, das vorzugsweise unter Chlorid, Methylsulfat und Acetat ausgewählt ist,
A eine Gruppe, die unter den folgenden Strukturen A1 bis A19 ausgewählt ist: 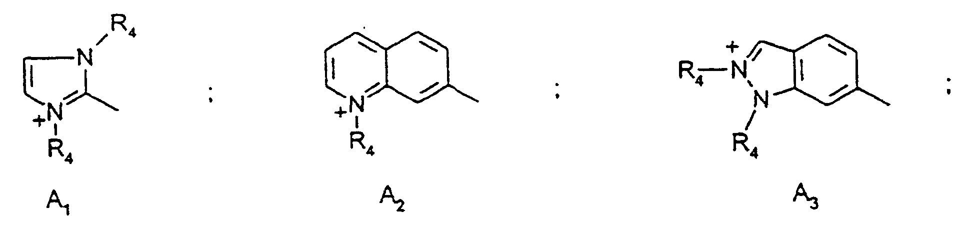 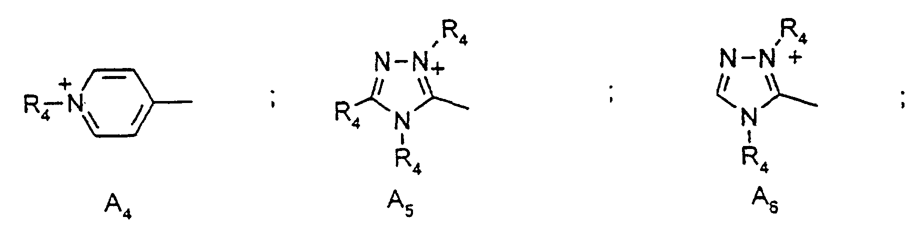 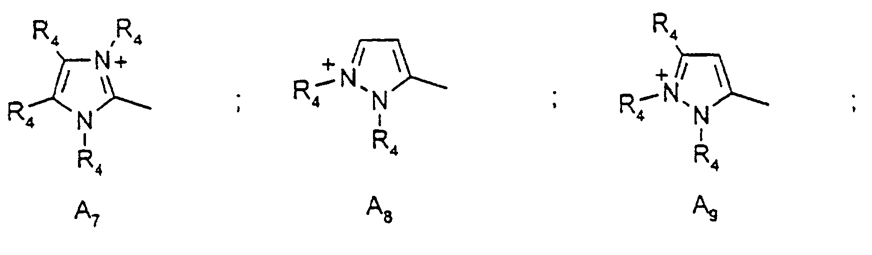 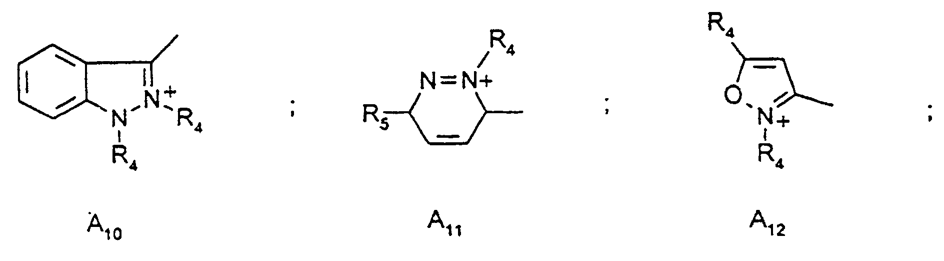 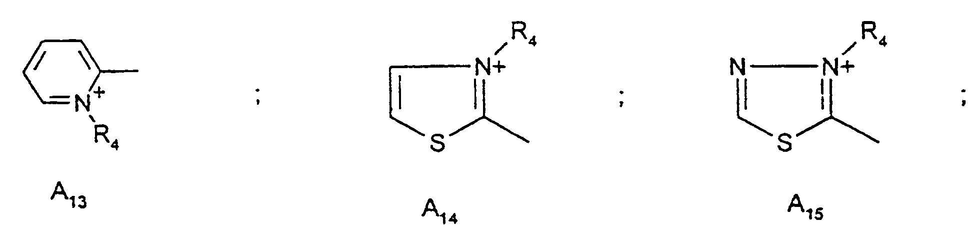 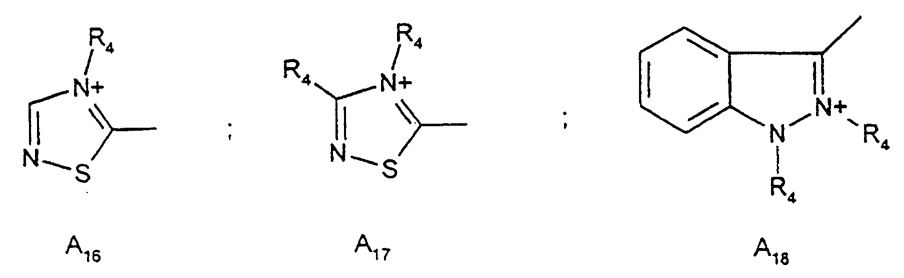 und 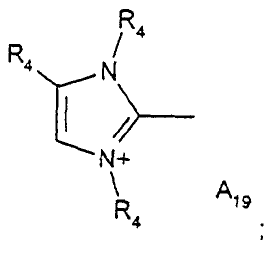 worin die Gruppe R₄ eine C₁₋₄-Alkylgruppe bedeutet, die mit einer Hydroxygruppe substituiert sein kann und R₅ eine C₁-₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
**b) Verbindungen der folgenden Formel (II):** 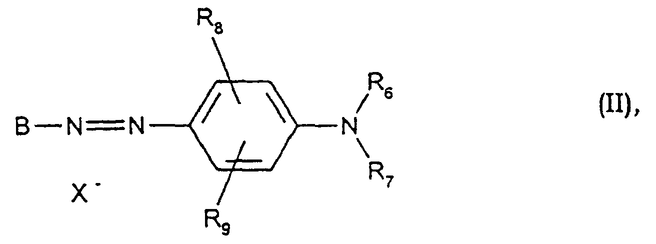 worin bedeuten:
R₆ ein Wasserstoffatom oder eine C₁₋₄-Alkylgruppe,
R₇ ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
die Gruppen R₈ und R₉, die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
X- ein Anion, das vorzugsweise unter Chlorid, Methylsulfat und Acetat ausgewählt ist,
B eine Gruppe, die unter den folgenden Strukturen B1 bis B6 ausgewählt ist: 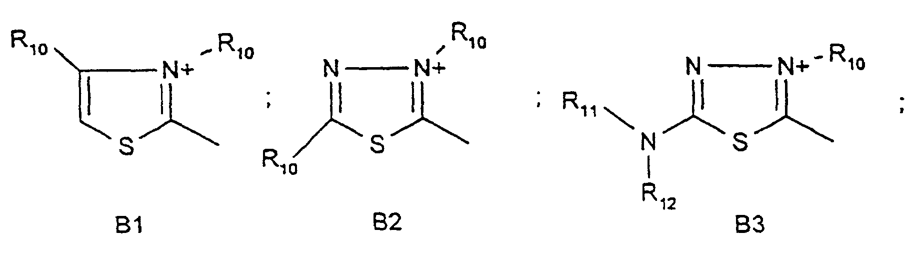 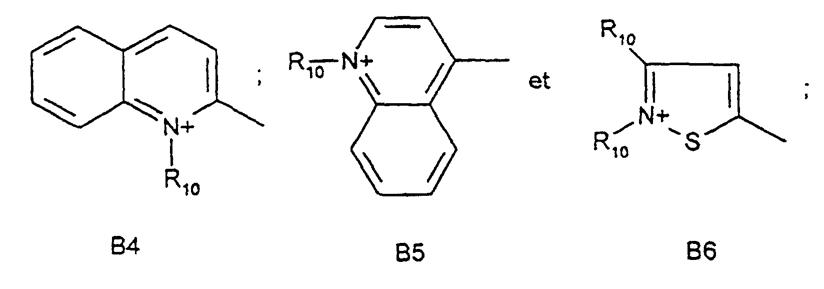 worin die Gruppe Rio eine C₁₋₄-Alkylgruppe bedeutet und die Gruppen R₁₁ und R₁₂, die gleich oder verschieden sind, Wasserstoff oder C₁₋₄-Alkyl bedeuten;
**c) Verbindungen der folgenden Formeln (III) und (III'):** 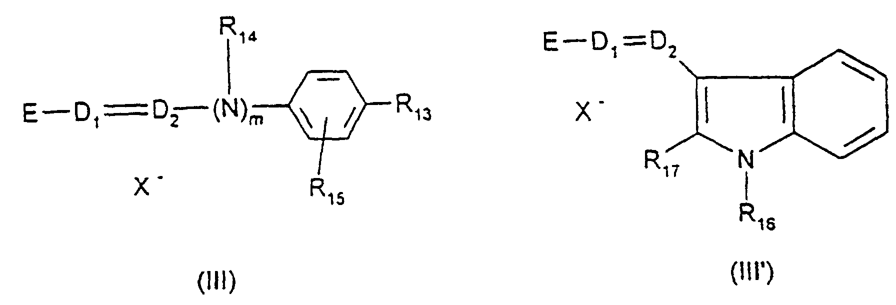 worin bedeuten:
R₁₃ ein Wasserstoffatom, eine C₁₋₄-Alkoxygruppe, ein Halogenatom, wie Brom, Chlor, Iod oder Fluor, oder eine Aminogruppe,
R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist; R₁₅ ein Wasserstoffatom oder ein Halogenatom, wie Brom, Chlor, Iod oder Fluor,
die Gruppen R₁₆ und R₁₇, die identisch oder voneinander verschieden sind, Wasserstoff oder C₁₋₄-Alkyl,
die Gruppen D₁ und D₂, die gleich oder verschieden sind, ein Stickstoffatom oder die Gruppe -CH,
m = 0 oder 1, mit der Maßgabe, dass die Gruppen D₁ und D₂ gleichzeitig -CH bedeuten und m = 0, wenn R₁₃ eine unsubstituierte Aminogruppe ist,
X⁻ ein Anion, das vorzugsweise unter Chlorid, Methylsulfat und Acetat ausgewählt ist,
E eine Gruppe, die unter den folgenden Strukturen E1 bis E8 ausgewählt ist: 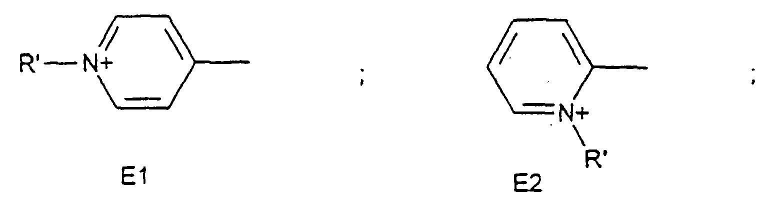 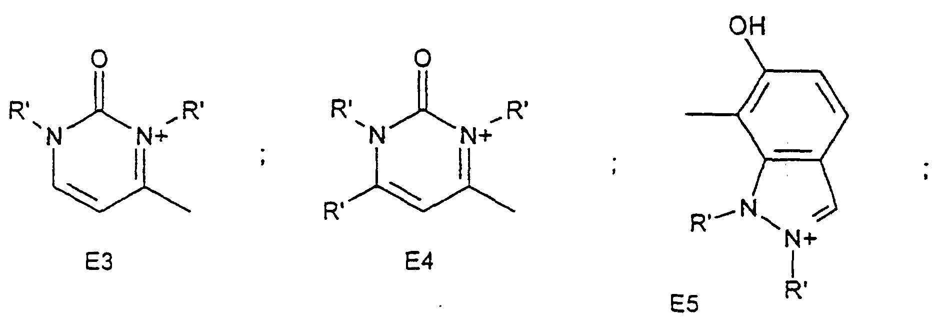 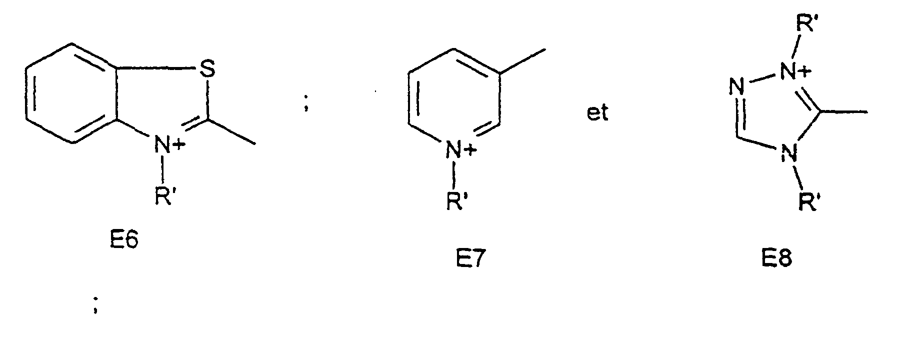 wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 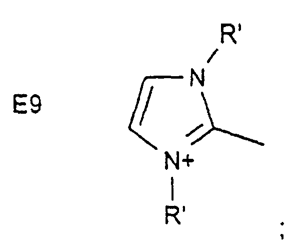 wobei R' eine C₁₋₄-Alkylgruppe bedeutet;
**d) Verbindungen der folgenden Formel (IV):**
G-N=N-J (IV),
worin bedeuten:
**das Symbol G** eine Gruppe, die unter den folgenden Strukturen G₁ bis G₃ ausgewählt ist: 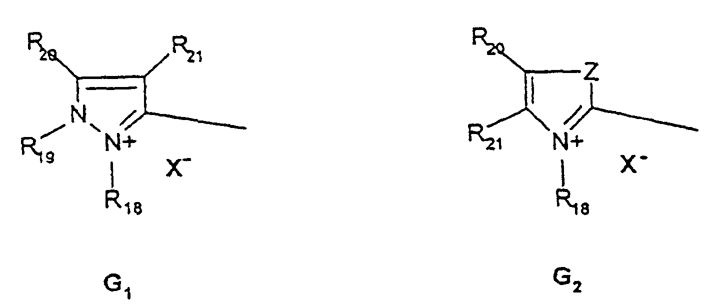 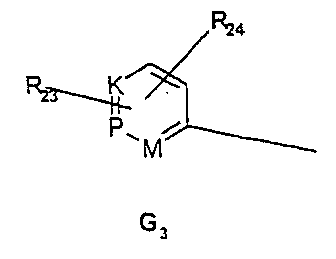 wobei in den Strukturen G₁ bis G₃ bedeuten:
R₁₈ C₁₋₄-Alkyl oder eine Phenylgruppe, die mit einer C₁₋₄-Alkylgruppe oder einem Halogenatom substituiert sein kann, das unter Chlor, Brom, Iod und Fluor ausgewählt ist;
R₁₉C₁₋₄-Alkyl oder Phenyl;
R₂₀ und R₂₁, die gleich oder verschieden sind, C₁₋₄-Alkyl, Phenyl oder R₂₀ und R₂₁ bilden in G₁ gemeinsam einen Benzolring, der mit einer oder mehreren Gruppen C₁₋₄-Alkyl, C₁₋₄-Alkoxy oder NO₂ substituiert ist, oder bilden in G₂ gemeinsam einen Benzolring, der gegebenenfalls mit einer oder mehreren G₁₋₄-Alkylgruppen, C₁₋₄-Alkoxy oder NO₂ substituiert ist;
wobei R₂₀ außerdem ein Wasserstoffatom bedeuten kann;
Z ein Sauerstoffatom, ein Schwefelatom oder eine Gruppe -NR₁₉;
M -CH, -CR (wobei R eine C₁₋₄-Alkylgruppe ist), oder - NR₂₂(X⁻)ᵣ;
K -CH, -CR (wobei R eine C₁₋₄-Alkylgruppe bedeutet) oder - NR₂₂(X⁻)ᵣ;
P eine Gruppe -CH, -CR (wobei R eine C₁₋₄-Alkylgruppe bedeutet) oder -NR₂₂(X⁻)ᵣ; wobei r Null oder 1 ist;
R₂₂ ein Atom O⁻, C₁₋₄-Alkoxy oder C₁₋₄-Alkyl;
R₂₃ und R₂₄, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X⁻ ein Anion, das vorzugsweise unter Chlorid, Iodid, Methylsulfat, Ethylsulfat, Acetat und Perchlorat ausgewählt ist;
mit der Maßgabe, dass
wenn R₂₂ O⁻ bedeutet, r Null ist;
wenn K oder P oder M -N(C₁-₄)alkyl X⁻ bedeutet, R₂₃ oder R₂₄ von Wasserstoff verschieden ist;
wenn K -NR₂₂(X⁻)ᵣ bedeutet, M = P = -CH, -CR;
wenn M -NR₂₂(X⁻)ᵣ bedeutet, K = P = -CH, -CR;
wenn P -NR₂₂(X⁻)ᵣ bedeutet, K = M = -CH oder -CR bedeutet; wenn Z ein Schwefelatom und R₂₁ C₁₋₄-Alkyl ist, R₂₀ von Wasserstoff verschieden ist;
wenn Z -NR₂₂ und R₁₉ C₁₋₄-Alkyl bedeutet, mindestens eine der Gruppen R₁₈, R₂₀ oder R₂₁ der Struktur G₂ von einer C₁₋₄-Alkylgruppe verschieden ist;
**das Symbol J:**
- **(a)** eine Gruppe der folgenden Struktur J₁: 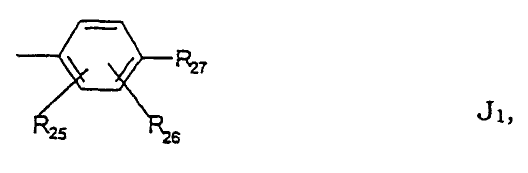 wobei in der Struktur J₁ bedeuten:
R₂₅ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₂₈, -NR₂₉R₃₀, -NHCOalkyl (C₁₋₄) oder R₂₅ bildet mit R₂₆ einen 5- oder 6-gliedrigen Ring, der gegebenenfalls ein oder mehrere Heteroatome enthält, die unter Stickstoff, Sauerstoff oder Schwefel ausgewählt sind;
R₂₆ ein Wasserstoffatom, ein Halogenatom, das unter Chlor, Brom, Iod und Fluor ausgewählt ist, C₁₋₄-Alkyl, C₁₋₄-Alkoxy oder R₂₆ bildet mit R₂₇ oder R₂₈ einen 5-oder 6-gliedrigen Ring, der gegebenenfalls ein oder mehrere Heteroatome enthält, die unter Stickstoff, Sauerstoff oder Schwefel ausgewählt sind;
R₂₇ Wasserstoff, -OH, -NHR₂₈ oder -NR₂₉R₃₀;
R₂₈ Wasserstoff, C₁₋₄-Alkyl, C₁₋₄-Monohydroxyalkyl, C₂₋₄-Polyhydroxyalkyl oder Phenyl;
R₂₉ und R₃₀, die gleich oder verschieden sind, C₁₋₄-Alkyl, C₁₋₄-Monohydroxyalkyl oder C₂₋₄-Polyhydroxyalkyl;
- **(b)** eine stickstoffhaltige, 5- oder 6-gliedrige heterocyclische Gruppe, die weitere Heteroatome und/oder carbonylhaltige Gruppen enthalten kann und mit einer oder mehreren Gruppen C₁₋₄-Alkyl, Amino oder Phenyl substituiert sein kann, insbesondere eine Gruppe der folgenden Struktur J₂: 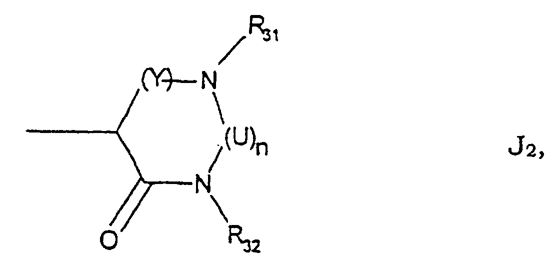 wobei in der Struktur J₂ bedeuten:
R₃₁ und R₃₂, die gleich oder verschieden sind, Wasserstoff, C₁₋₄-Alkyl oder Phenyl;
Y die Gruppe -CO- oder die Gruppe 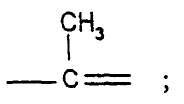
n = 0 oder 1, wobei U die Gruppe -CO- bedeutet, wenn n 1 ist.

14. Verfahren nach einem der vorhergehenden Ansprüche, **dadurch gekennzeichnet, dass** das wässrige Medium der Farbmittelzusammensetzung ausschließlich aus Wasser oder einem Gemisch von Wasser und mindestens einem kosmetischen Zusatzstoff besteht, der unter den Lösungsmitteln, grenzflächenaktiven Stoffen, Polymeren, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Ceramiden, Konservierungsmitteln und Trübungsmitteln ausgewählt ist.

15. Verfahren nach einem der vorhergehenden Ansprüche, **dadurch gekennzeichnet, dass** das Wasser in einer Menge von 20 bis 95 Gew.-% des Gesamtgewichts der Farbmittelzusammensetzung vorliegt.

16. Verfahren nach einem der vorhergehenden Ansprüche, **dadurch gekennzeichnet, dass** in der pulverförmigen Zusammensetzung (P) der oder die basischen Direktfarbstoffe die gesamte pulverförmige Zusammensetzung ausmachen oder in einem pulverförmigen Träger organischer und/oder anorganischer Natur dispergiert sind.

17. Verfahren nach Anspruch 16, **dadurch gekennzeichnet, dass** es sich bei dem Träger um Sägemehl handelt.

18. Verfahren nach einem der vorhergehenden Ansprüche, **dadurch gekennzeichnet, dass** die Farbmittelzusammensetzung während einer Einwirkzeit von 2 bis 45 min aufgebracht wird.

19. Verfahren nach Anspruch 18, **dadurch gekennzeichnet, dass** die Einwirkzeit im Bereich von 3 bis 10 min liegt.

## Claims

1. Process for the two-step direct dyeing of keratin fibres, and in particular human keratin fibres such as the hair, **characterized in that**, in a first step, the keratin fibres are bleached, and, in a second step, a dye composition containing, in an aqueous medium, at least one partially dissolved basic direct dye is then applied to the keratin fibres, the said dye composition being ready to use or resulting from the mixing, at the time of use, of at least one pulverulent composition (P) containing at least one basic direct dye and an aqueous composition (A).

2. Process according to Claim 1, **characterized in that** the basic direct dye(s) is (are) in supersaturation.

3. Process according to Claim 1, **characterized in that** the basic direct dye(s) is (are) partially or totally adsorbed onto or coated with an insoluble inorganic or organic filler present in the dye composition.

4. Process according to any one of the preceding claims, **characterized in that** the first step is a bleaching step which leads to a shade having a tone height of greater than or equal to 6.

5. Process according to Claim 4, **characterized in that** the bleaching step leads to a shade which shows a colour difference of greater than or equal to 2 tones when compared with the colour of the fibres before bleaching.

6. Process according to any one of the preceding claims, **characterized in that** the bleaching is carried out by applying an oxidizing composition containing at least one oxidizing agent.

7. Process according to Claim 6, **characterized in that** the oxidizing agent is chosen from hydrogen peroxide, urea peroxide, alkali metal bromates, persalts and polythionates, and mixtures thereof.

8. Process according to either of Claims 6 and 7, **characterized in that** the oxidizing composition is in liquid or creamy form, the said oxidizing composition being ready to use or resulting from the mixing, at the time of use, of one or more aqueous compositions or of one or more aqueous compositions with one or more pulverulent compositions, the oxidizing agent being present in the aqueous composition(s) and/or in the pulverulent composition(s).

9. Process according to any one of Claims 6 to 8, **characterized in that** the oxidizing composition has a pH of between 5 and 12.

10. Process according to any one of Claims 1 to 5, **characterized in that** the bleaching is carried out by irradiating the keratin fibres with laser light in the form of pulses which are powerful enough to degrade the melanin contained in the keratin fibres.

11. Process according to any one of the preceding claims, **characterized in that** the basic direct dye(s) is (are) chosen from basic aminoanthraquinone dyes, basic mono- or diazo dyes, basic azomethine dyes, basic naphthoquinone dyes and basic dyes containing ethylenic monounsaturations.

12. Process according to Claim 11, **characterized in that** the dyes are chosen from [8-[(p-aminophenyl)azo]-7-hydroxy-2-naphthyl]trimethylammonium chloride (also known as Basic Brown 16 or Arianor Mahogany 306002 in the Color Index), the combination of 3-[(4-amino-6-bromo-5,8-dihydro-1-hydroxy-8-imino-5-oxo-2-naphthyl)amino]-N,N,N-trimethylbenzenaminium chloride and of 3-[(2,6-dibromo-5,8-dihydro-1-hydroxy-8-imino-5-oxo-3-naphthyl)amino]-N,N,N-trimethylbenzenaminium chloride (also known as Basic Blue 99 or Arianor Steel Blue 306004 in the Color Index), 7-hydroxy-8-[(2-methoxyphenyl)azo]-N,N,N-trimethyl-2-naphthalenaminium chloride (also known as Basic Red 76 or Arianor Madder Red in the Color Index), [8-[(4-amino-2-nitrophenyl)azo]-7-hydroxy-2-naphthyl]trimethylammonium chloride (also known as Basic Red 118 in the Color Index), the combination of [8-[(4-amino-3-nitrophenyl)azo]-7-hydroxy-2-naphthyl)trimethylammonium chloride and of [8-[(4-amino-2-nitrophenyl)azo]-7-hydroxy-2-naphthyl]-trimethylammonium chloride (also known as Brown 17 or Arianor Sienna Brown 306001 in the Color Index), 3-[(4,5-dihydro-3-methyl-5-oxo-l-phenyl-1H-pyrazol-4-yl)azo]-N,N,N-trimethylbenzenaminium chloride (also known as Basic Yellow 57 or Arianor Straw Yellow 306005 in the Color Index), 1-(γ-aminopropyl)aminoanthraquinone hydrochloride, 1-N-(methylmorpholiniumpropyl)amino-4-hydroxyanthraquinone methyl sulphate and Basic Orange 69 (Color Index name).

13. Process according to any one of Claims 1 to 10, **characterized in that** the basic direct dye(s) is (are) chosen from:
**a) the compounds of formula (I) below:** 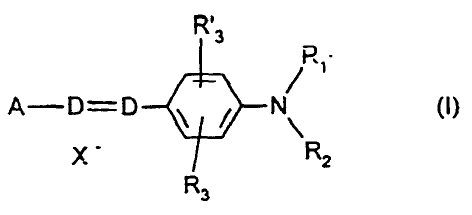 in which:
D represents a nitrogen atom or a -CH group,
R₁ and R₂, which may be identical or different, represent a hydrogen atom; a C₁-C₄ alkyl radical which can be substituted with a -CN, -OH or -NH₂ radical or form, with a carbon atom of the benzene ring, a heterocycle optionally containing oxygen or nitrogen, which can be substituted with one or more C₁-C₄ alkyl radicals; a 4'-aminophenyl radical,
R₃ and R'₃, which may be identical or different, represent a hydrogen atom or a halogen atom chosen from chlorine, bromine, iodine and fluorine, or a cyano, C₁-C₄ alkoxy or acetyloxy radical,
X⁻ represents an anion preferably chosen from chloride, methyl sulphate and acetate,
A represents a group chosen from structures A₁ to A₁₉ below : 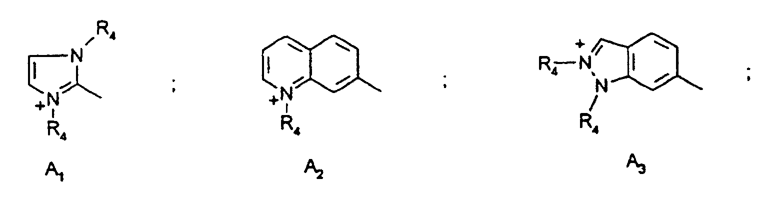 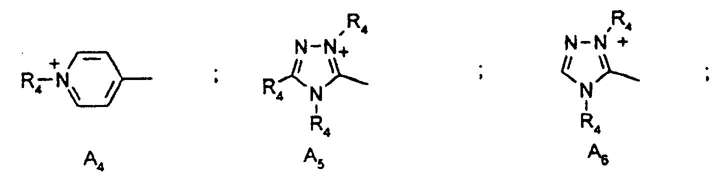 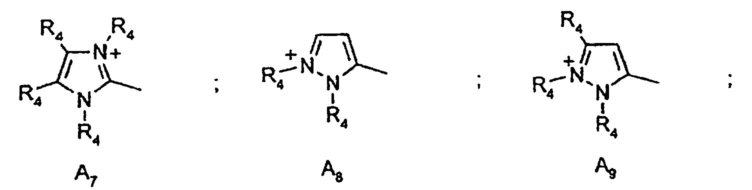 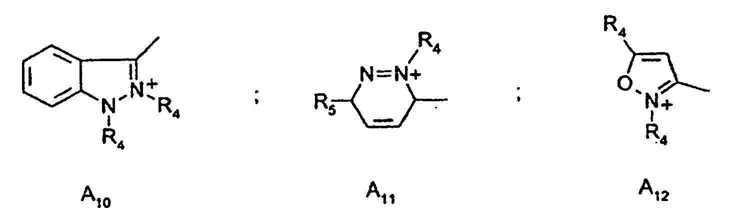 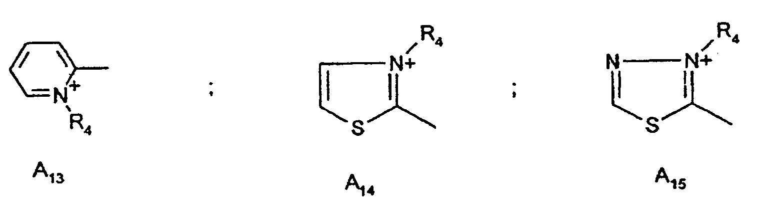 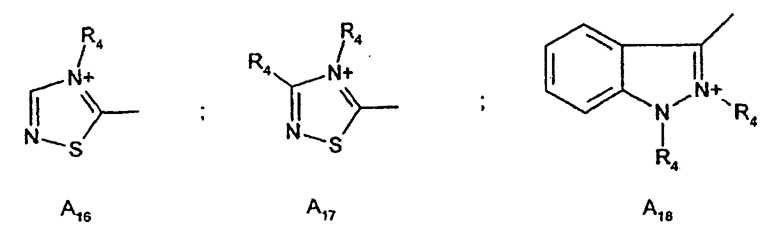 and 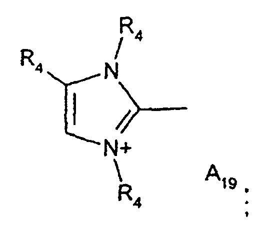 in which R₄ represents a C₁-C₄ alkyl radical which can be substituted with a hydroxyl radical and R₅ represents a C₁-C₄ alkoxy radical, with the proviso that when D represents -CH, A represents A₄ or A₁₃ and R₃ is other than an alkoxy radical, then R₁ and R₂ do not simultaneously denote a hydrogen atom;
**b) the compounds of formula (II) below:** 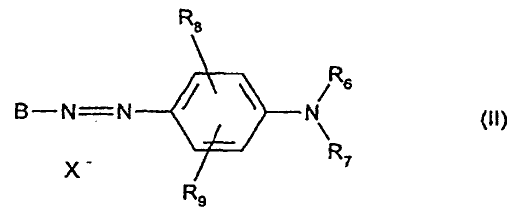 in which:
R₆ represents a hydrogen atom or a C₁-C₄ alkyl radical,
R₇ represents a hydrogen atom, an alkyl radical which can be substituted with a -CN radical or with an amino group, a 4'-aminophenyl radical or forms with R₆ a heterocycle, optionally containing oxygen and/or nitrogen, which can be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methyl sulphate and acetate,
B represents a group chosen from the structures B1 to B6 below: 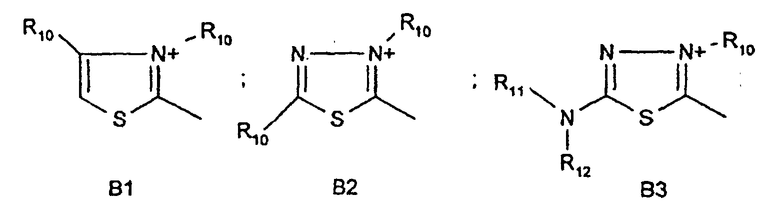 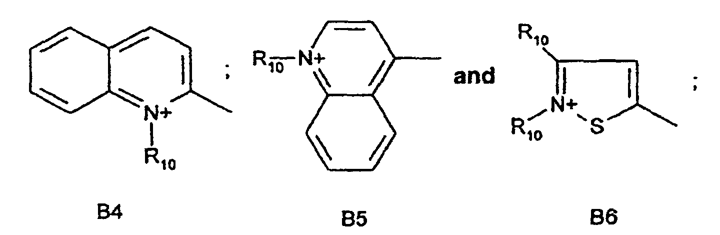 in which R₁₀ represents a C₁-C₄ alkyl radical and R₁₁ and R₁₂, which may be identical or different, represent a hydrogen atom or a C₁-C₄ alkyl radical;
**c) the compounds of formulae (III) and (III') below:** 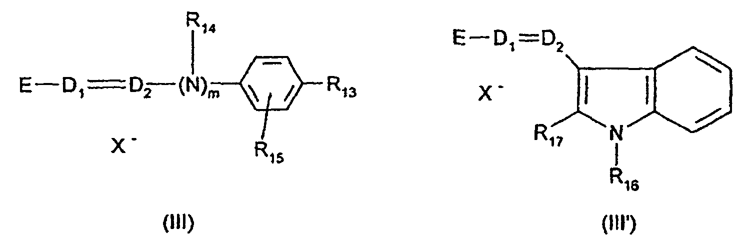 in which:
R₁₃ represents a hydrogen atom, a C₁-C₄ alkoxy radical, a halogen atom such as bromine, chlorine, iodine or fluorine or an amino radical,
R₁₄ represents a hydrogen atom, a C₁-C₄ alkyl radical or forms, with a carbon atom of the benzene ring, a heterocycle optionally containing oxygen and/or substituted with one or more C₁-C₄ alkyl groups,
R₁₅ represents a hydrogen atom or a halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methyl sulphate and acetate,
E represents a group chosen from the structures E1 to E8 below: 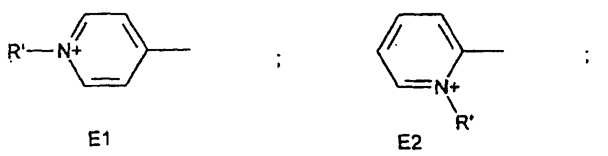 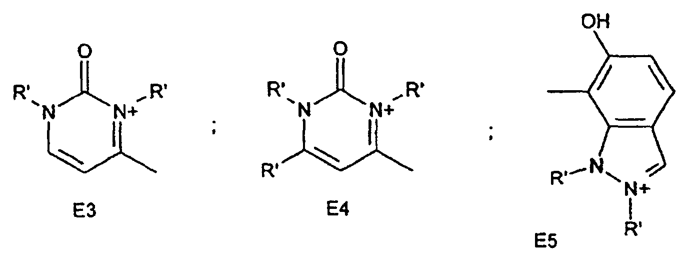 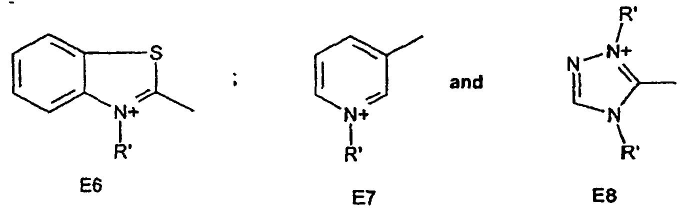 in which R' represents a C₁-C₄ alkyl radical; when m = 0 and D₁ represents a nitrogen atom, then E can also denote a group of structure E9 below: 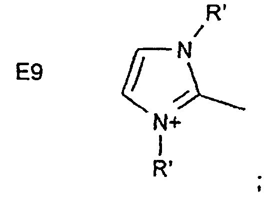 in which R' represents a C₁-C₄ alkyl radical;
**d) the compounds of formula (IV) below:**
**G―N ̿N―J (IV)**
in which:
the symbol G represents a group chosen from the structures G₁ to G₃ below: 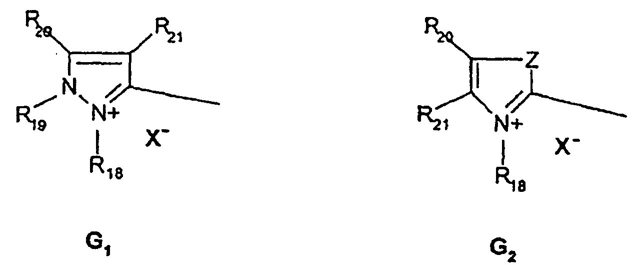 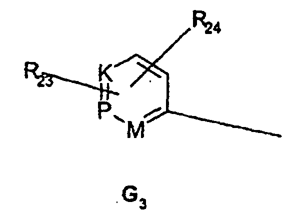 in which structures G₁ to G₃:
R₁₈ denotes a C₁-C₄ alkyl radical, a phenyl radical which can be substituted with a C₁-C₄ alkyl radical or a halogen atom chosen from chlorine, bromine, iodine and fluorine;
R₁₉ denotes a C₁-C₄ alkyl radical or a phenyl radical;
R₂₀ and R₂₁, which may be identical or different, represent a C₁-C₄ alkyl radical, a phenyl radical or together form, in G₁, a benzene ring substituted with one or more C₁-C₄ alkyl, C₁-C₄ alkoxy or NO₂ radicals, or together form, in G₂, a benzene ring optionally substituted with one or more C₁-C₄ alkyl, C₁-C₄ alkoxy or NO₂ radicals;
R₂₀ can also denote a hydrogen atom;
Z denotes an oxygen or sulphur atom or a group -NR₁₉;
M represents a -CH, -CR (R denoting C₁-C₄ alkyl) or -NR₂₂(X⁻)ᵣ group;
K represents a -CH, -CR (R denoting C₁-C₄ alkyl) or -NR₂₂(X⁻)ᵣ group;
P represents a -CH, -CR (R denoting C₁-C₄ alkyl) or -NR₂₂(X⁻)ᵣ group; r denotes zero or 1;
R₂₂ represents an atom O⁻, a C₁-C₄ alkoxy radical or a C₁-C₉ alkyl radical;
R₂₃ and R₂₄, which may be identical or different, represent a hydrogen atom, a halogen atom chosen from chlorine, bromine, iodine and fluorine, a C₁-C₄ alkyl or C₁-C₄ alkoxy radical or an -NO₂ radical;
X⁻ represents an anion preferably chosen from chloride, iodide, methyl sulphate, ethyl sulphate, acetate and perchlorate;
with the proviso that,
if R₂₂ denotes O⁻, then r denotes zero;
if K or P or M denotes -N-(C₁-C₄)alkyl X⁻, then R₂₃ or R₂₄ is other than a hydrogen atom;
if K denotes -NR₂₂(X⁻)ᵣ, then M=P=-CH or -CR;
if M denotes -NR₂₂(X⁻)ᵣ, then K=P=-CH or -CR;
if P denotes -NR₂₂(X⁻)ᵣ, then K=M and denote -CH or - CR;
if Z denotes a sulphur atom with R₂₁ denoting C₁-C₄ alkyl, then R₂₀ is other than a hydrogen atom;
if Z denotes -NR₂₂ with R₁₉ denoting C₁-C₄ alkyl, then at least one of the radicals R₁₈, R₂₀ or R₂₁ of G₂ is other than a C₁-C₄ alkyl radical;
the symbol J represents:
- (a) a group of structure J₁ below: 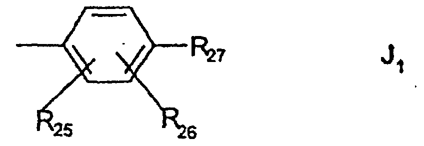 in which structure J₁:
R₂₅ represents a hydrogen atom, a halogen atom chosen from chlorine, bromine, iodine and fluorine, a C₁-C₄ alkyl or C₁-C₄ alkoxy radical, a radical -OH, -NO₂, -NHR₂₈, -NR₂₉R₃₀, or -NHCO(C₁-C₄)alkyl, or forms, with R₂₆, a 5- or 6-membered ring which may or may not contain one or more hetero atoms chosen from nitrogen, oxygen and sulphur;
R₂₆ represents a hydrogen atom, a halogen atom chosen from chlorine, bromine, iodine and fluorine, a C₁-C₄ alkyl or C₁-C₄ alkoxy radical, or forms, with R₂₇ or R₂₈, a 5- or 6- membered ring which may or may not contain one or more hetero atoms chosen from nitrogen, oxygen and sulphur;
R₂₇ represents a hydrogen atom, an -OH radical, an -NHR₂₈ radical or an -NR₂₉R₃₀ radical;
R₂₈ represents a hydrogen atom, a C₁-C₄ alkyl radical, a C₁-C₄ monohydroxyalkyl radical, a C₂-C₄ polyhydroxyalkyl radical or a phenyl radical;
R₂₉ and R₃₀, which may be identical or different, represent a C₁-C₄ alkyl radical, a C₁-C₄ monohydroxyalkyl radical or a C₂-C₄ polyhydroxyalkyl radical;
- (b) a 5- or 6-membered heterocyclic group containing nitrogen, which can contain other hetero atoms and/or carbonyl groups and can be substituted with one or more C₁-C₄ alkyl, amino or phenyl radicals, and in particular a group of structure J₂ below: 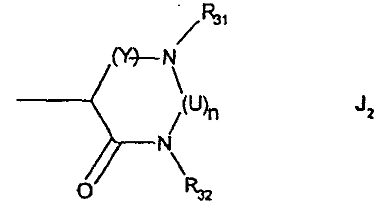 in which structure J₂:
R₃₁ and R₃₂, which may be identical or different, represent a hydrogen atom, a C₁-C₄ alkyl radical or a phenyl radical;
Y denotes a -CO- radical or a radical 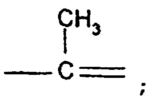 n = 0 or 1 with, when n denotes 1, U denoting a - CO- radical.

14. Process according to any one of the preceding claims, **characterized in that** the aqueous medium of the dye composition consists solely of water or of a mixture of water and at least one cosmetic adjuvant chosen from solvents, surfactants, polymers, inorganic or organic thickeners, antioxidants, penetrating agents, sequestering agents, fragrances, buffers, dispersing agents, conditioners, film-forming agents, ceramides, preserving agents and opacifiers.

15. Process according to any one of the preceding claims, **characterized in that** the water represents from 20 to 95% by weight of the total weight of the dye composition.

16. Process according to any one of the preceding claims, **characterized in that**, in the pulverulent composition (P), the basic direct dye(s) constitute, by themselves, all of the pulverulent composition, or are dispersed in an excipient, as powder, of organic nature and/or of inorganic nature.

17. Process according to Claim 16, **characterized in that** the excipient is sawdust.

18. Process according to any one of the preceding claims, **characterized in that** the dye composition is applied for a dye-application time of between 2 and 45 minutes.

19. Process according to Claim 18, **characterized in that** the dye-application time is between 3 and 10 minutes.
